# EUROPEAN PATENT SPECIFICATION

(11) **EP 0 671 909 B1**
(45) Date of publication and mention of the grant of the patent: **27.12.2000**
(21) Application number: 94900846.0
(22) Date of filing: 01.12.1993
(51) Int. Cl.: A61K 31/135, A61K 38/00

(54) **SYNDECAN STIMULATION OF CELLULAR DIFFERENTIATION**
SYNDECAN-STIMULIERUNG DER ZELLULÄREN DIFFERENZIERUNG
STIMULATION DE LA DIFFERENCIATION CELLULAIRE PAR SYNDECANE

(30) Priority: 01.12.1992 US 988427
(43) Date of publication of application: 20.09.1995
(73) Proprietor: Oy Biotie Therapies, 20520 TURKU (FI)
(72) Inventor: Wärri, Anni Maija, 21420 Lieto (FI); Alanen-Kurki, Leena Maria, 20500 Turku (FI); Auvinen, Petri Olli Viljami, 69126 Heidelberg (DE); Jaakola, Panu Mikko, 20500 Turku (FI); Jalkanen, Markku Tapani, 20760 Piispanristi (FI); Leppä, Sirpa Marianne, 20660 Littoinen (FI); Mali, Markku Sakari, 24280 Salo (FI); Vihinen, Tapani Antero, 20700 Turku (FI)
(74) Representative: VOSSIUS & PARTNER
(86) International application number: FI9300514
(87) International publication number: WO9412162

(56) References cited:
- EP-A- 0 335 554
- EP-A- 0 455 422
- EP-A- 0 462 398
- WO-A-92/13274
- CELL REGULATION vol. 2 , 1991 pages 1 - 11 S. LEPPA ET AL. 'STEROID-INDUCED EPITHELIAL-FIBROBLASTIC CONVERSION ASSOCIATED WITH SYNDECAN SUPPRESSION IN S115 MOUSE MAMMARY TUMOR CELLS' cited in the application
- PROC. NATL. ACAD. SCI. vol. 89, no. 3 , February 1992 pages 932 - 936 S. LEPPA ET AL. 'SYNDECAN EXPRESSION REGULATES CELL MORPHOLOGY AND GROWTH OF MOUSE MAMMARY EPITHELIAL TUMOR CELLS' cited in the application
- BIOCHEMICAL SOCIETY TRANSACTIONS vol. 19, no. 4 , 1991 pages 1069 - 1072 M. JALKANEN ET AL. 'SYNDECAN, A REGULATOR OF CELL BEHAVIOUR, IS LOST IN MALIGNANT TRANSFORMATION'
- JOURNAL OF CELLULAR BIOCHEMISTRY vol. SUPPL., no. 15F , 1991 page 223 M. JALKANEN ET AL. 'STIMULATION OF SYNDECAN GENE EXPRESSION IN MESENCHYMAL CELLS BY bFGF AND TGF-beta'
- THE JOURNAL OF BIOLOGICAL CHEMISTRY vol. 267, no. 9 , March 1992 pages 6435 - 6441 K. ELENIUS ET AL. 'GROWTH FACTORS INDUCE 3T3 CELLS TO EXPRESS bFGF-BINDING SYNDECAN' cited in the application
- THE JOURNAL OF CELL BIOLOGY vol. 114, no. 3 , 1991 pages 585 - 595 K. ELENIUS ET AL. 'INDUCED EXPRESSION OF SYNDECAN IN HEALING WOUNDS'
- J.E.F. REYNOLDS 'MARTINDALE,THE EXTRA PHARMACOPOEIA' 1989 , THE PHARMACEUTICAL PRESS , LONDON see page 650 - page 651 see page 1625
- THE JOURNAL OF CLINICAL INVESTIGATION vol. 80, no. 5 , 1987 pages 1516 - 1520 L. SCHWEIGERER ET AL. 'BASIC FIBROBLAST GROWTH FACTOR AS A GROWTH INHIBITOR FOR CULTURED HUMAN TUMOR CELLS'

SEQUENCE LISTING

(1) GENERAL INFORMATION:

(i) APPLICANT:
(A) NAME: Alanen-Kurki, Leena
(B) STREET: Piispankatu 6 E 31
(C) CITY: TURKU
(E) COUNTRY: FINLAND
(F) POSTAL CODE (ZIP): FIN-20500

(A) NAME: Auvinen, Petri
(B) STREET: Kirschgartenstrasse 5
(C) CITY: HEIDELBERG
(E) COUNTRY: GERMANY
(F) POSTAL CODE (ZIP): D-69126

(A) NAME: Jaakkola, Panu
(B) STREET: Kellonsoittajankatu 13 B 20
(C) CITY: TURKU
(E) COUNTRY: FINLAND
(F) POSTAL CODE (ZIP): FIN-20500

(A) NAME: Jalkanen, Markku
(B) STREET: Rauvolantie
(C) CITY: PIISPANRISTI
(E) COUNTRY: FINLAND
(F) POSTAL CODE (ZIP): FIN-20760

(A) NAME: Leppa, Sirpa
(B) STREET: Valkintie 14 as 6
(C) CITY: LITTOINEN
(E) COUNTRY: FINLAND
(F) POSTAL CODE (ZIP): FIN-20660

(A) NAME: Mali, Markku
(B) STREET: Inkereentie 176
(C) CITY: SALO
(E) COUNTRY: FINLAND
(F) POSTAL CODE (ZIP): FIN-24280

(A) NAME: Vihinen, Tapani
(B) STREET: Kaskenkatu 11 C 54
(C) CITY: TURKU
(E) COUNTRY: FINLAND
(F) POSTAL CODE (ZIP): FIN-20700

(A) NAME: Warri, Anni
(B) STREET: Juhaninkuja 2 as 2
(C) CITY: LIETO
(E) COUNTRY: FINLAND
(F) POSTAL CODE (ZIP): FIN-21420

(ii) TITLE OF INVENTION: SYNDECAN STIMULATION OF CELLULAR
DIFFERENTIATION

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: WO TO BE ASSIGNED
(vi) PRIOR APPLICATION DATA:
(A) APPLICATION NUMBER: US 07/988427
(B) FILING DATE: 01-DEC-1992

(2) INFORMATION FOR SEQ ID NO: 1:

(i) SEQUENCE CHARACTERISTICS:
(A) LENGTH: 26700 base pairs
(B) TYPE: nucleic acid
(C) STRANDEDNESS: both
(D) TOPOLOGY: linear

(ii) MOLECULE TYPE: DNA (genomic)

(iii) HYPOTHETICAL: NO

(iii) ANTI-SENSE: NO

(ix) FEATURE:
(A) NAME/KEY: CDS
(B) LOCATION: join(4378..4443, 22026..22106, 23001..23483,
23905..24039, 24251..24418)

(xi) SEQUENCE DESCRIPTION: SEQ ID NO: 1:

TCTAGATATT CAAACTCACC AGATGGAGTG ATGTCCACCC CTATTGGTGG GAGTGACTAG 60

TCTTTCCTCT GTCTTCTGAC TCAGATGCTT AGCTAGCTCT TTAGGACCCA CCCTCACACC 120

TGCAAATAAT ACTTTATTTG CTCTCTTAGT ACCTTTAACC CAGTGGAGTT GACATGAGAA 180

ATTAACTACC ATAATTTATA ATATTTCATT TCATAAATGA AAAGTAAAAT AAATTAAAAA 240

ATAGAAAGGT TTGAGCATGA TGGCCCAGTG GTAAAGGCCA GTGGCTCCAA CGCAAGTCCT 300

GACAAATGGT AACGGGCCTG TTCTTCAGGC TTGAGGGAAG TTTATTGATT GAGGCTAAAA 360

GCAACCCAAA GGCTCCACTT GCCTAGTGTG AAGCCCTGGA TGTGCTCTCC CACACTGCAT 420

GTCCACCTGT GGTGTCAGCA CCTGGGAAGC TGAGGATGAT GGGGAGTCCA AGGTCATTAG 480

CTACATAGTA TAGGCTAGCT GGGGTACATG GGTCACAAAA AAGAAAAAAA AATAAGCACA 540

TTGTAATCCC AGCACTTGAC AGACCAATGG GGGGGGGATT GCTGTGAGTT TAAGACAGCC 600

TGGCCTACAA AGAAAAACCC TACCCAAACC CAAGAAAAAT GAAACCAGTA ATATAAATAG 660
CTATTTTCAT TTTAAATGCT CTAAAGACAC AGCGTTAACA CAAAAGCTCT CGTCTGTGGT 720
TCCTATTCCC TCCTTCTCCC CCAGGTCTTC TTTAATGTAT ACTTTTTGTT TGCTTATTTG 780
CTTGTTTTGG ATTTTGGCTT TTAAAGACAG GGTCTCACTA TGTAGCTCCA ACTATTTGGG 840
AACTCACTAT GTAGACCAGG CTAGCCAGGG ACTTATAGAG ATCTACCTAC CACTGCCTCC 900
CAAGTGCTGA GACTAAAGGC ATGTGACACT TTGCTTGGTT ATTACAAACA TTTTAAAAGA 960
ACATTTTGAA CATTAATAGA TGTATGTATA TATATCACTC TATGTAGTAT ATATGTTAGA 1020
CATTTTTCAC TTGAGATACA TATTTACTCT CAAAATAAGT TTTTTGTTTT TTTTTCTTCT 1080
TTTTAAATTT ATTTTATTTT TTTTTTATTT ATTTTATTAT TATATGTAAG TACACTGTAG 1140
CTGTCTTCAG ACANACCAGA AGAGGGAGTC AGATCTTGTT ACGGATGGTT GTGAGCACCA 1200
TGTGGTTGCT GGGATTCGAA CTCTGGACCT TCCGAAGAGC AGTCGGGTGC TCTTACCCAC 1260
TGAGCCATCT CACCAGCCCC TTAAATTTAT TTTTATCTTA TGTCCATTGG TGTTTTGCCT 1320
GCATGTATGT GTAAAAGTGT CAGAAACTGA AGTTACAGAC TGTTGTGAGC TACCATTGTT 1380
GTGGGTGCTG GGACTTGAAC CTGGGTCCTC TGGAAGAGCA GTCATTATTC TTAACCACTG 1440
AGCCATCTCT CTAGCCCTCG TTTTTTAGTT TTTTTTTTTG TTTTGTTTTG TTTTTTGTTT 1500
TTTTAAGATT TTCTTATTTA TTATATGTAA GTACACTGTA GCTGTCTTCA GACACTCCAG 1560
AAGAGGGCGC CAGATCTCGT TATGGATGGT TGTGAGCACC ATGTGGTTGC TGGGAATTGA 1620
ACTCCAGACC TTTGGAAGAG CAGTCAGTGC TCTTAACTGC TGAGCCATCT CTCCAGCCCC 1680
GTTTTTTAGG TTTTTGAAGA CAGGGTTTCC TGTGTAGCTC TAGCTGTCCA GGAACTAGCT 1740
CTGTAGACCA GGTTGGCCTC AAATTTAGAG ATTTGCCTGT CTCTCTGCCT CTCGAGAGCT 1800
GGGATTAAAA GTGTGCAGCC CAACAATCTA CTCAAAGTAG GTTTTGAAAA AGCTTTCCAT 1860
ATTAGGAGTT AACTAGCTTC ATTTCAGAAA TACTGCATGG AATTCAAATG TGGGACCATT 1920
CATAGCTACT TTGGTTTTCC TTCAGTGACA GGCATTCGGC ATGCCTATTA GGGAAGTCAA 1980
ATGGCCTGGA GAAGTCATCC TGGGTGAGAG GGCTAATGCA TTTTCAGCTT GACAGACACT 2040
GTCAACCTAT GCAGACAGTC TGCTCCAGCT CAGATGTCAA TTGCATGCAG ACCTGCAGTC 2100
AGACGCTAAG CTCCCTACCT ACTCTCCATC AGCTTAGATG TAAGGGGTGC TGGAACAAAG 2160
GCTCTCTCTC TCTCTCTCTC TCTCTCTCTC TCTCTCTCTT TCTTAGAATT AGTATTCTAT 2220
TTTATTTTAT GTAAATTGGT ACTTCACTTA CATGTATGTC CGTGTGAGGA TGTTGTATCC 2280
TCTGGTACTG GAGTTATAGA CAGCTGTAAG TCGCCATACA GGTGCTGGGA ATTGAACCCT 2340

GATCCTCTGG AAGAATAGTC AGTGCTCTTA ACCCCTGAGC CATCTCTCCA ACCTCTTGCA 2400
TATTGAGGAC AGGGAGGAAT CACAAGCCAT GTAGGGTGCC TGGGCTCTGA GGTCAACAGG 2460
ACCATAGCCT CCTTTCTTTA TGTGCCTTTC TTGGGGTCTC CCTATAGGAG TCGTCTTCGT 2520
TGCCTCTTTA CTGTCTCATT GATCTGGGCT AAACTTATGC AGTTGGAAGG AAAGATCAAG 2580
CTGGTCATGT TTAAAACATG AAACAGCCTC ATCAGTTCCC TTCCTGTTCC CGTCTCCCCC 2640
CCCCCTCCCG CCCCCATTTT GAGAGGACAG GAAGGTAAAA TACCAAAGTG TCCTATTTTC 2700
CTCCAAATAT CAGGCTCAAA GGACTGAAGA GCTGACTTCA GATCCCAAAG CCACTGTGTT 2760
AGGAGGCACC TGCTTTTTAG GTCCTAAGCC TTCCTGAGCC TTGCTATTGG GTATTCTTTA 2820
CCAAGACCCT CAAGGATCTA GGCAAGAACT GGGCAGGATC TGTATGTAGC CCATAGTTAG 2880
ACCTAGGGCA GCTGAGACGC CAAAAGGGAG AGTTTCCTGA GGACAAAAGT GTTCAAACAC 2940
AACTGGGTGC TGGTTGTTGG GCTACTCGTG GAGGTGTGGT GTGTGTAAAG GAGGCTGTTG 3000
AATTCCCAGA AGGCTGGTTC CACAGTGTAG AGTCTACACT GGGGACTTCC CGAGACGCTG 3060
AGCCTCAGAT CTAGCTTCTC AGTCCAGGCC AGCTGATGTG GGGCTGAGGA ACAAGGATGG 3120
ATGCCATCTA TGGCCCTGCC TTGCAGGTGC AAAGGGCCTT TGGCACCATC TACAGATTGA 3180
GGGCAAGACA GGGCTGGTTC TTCCTCCTTG CTCTCGCTGC TATCTGCCTC GCCTGTAGGC 3240
TCTCTGGGCT CCTTTTTGGA CTGACACGTC TGAAGGAGCT TGGAAACTGT GAGGTCCAGG 3300
CCCCATAGAG AATCATGAAG GAACAGGAAT TCAACTGGAG CTCCGCAGCT GGTTAGGCCT 3360
GCGGTCACCT GGAAACAAAG AGGCCATTTA TTTTTTCCTT TGGTCTTGGA CAAGGAAGAG 3420
AAGGGGCTTT CTATAAATAG AAAGACAGCA AAAAAGAAAA TAATAATAAT AATAATAATA 3480
ATAATAATAA TAATAAAAAC AATAACAAAG CCAGCTCTTC CAGACAGTGC TCATGTCTTT 3540
AAAGGTCTTT AAAGGTCTGG AGTTCCCAGC AATTAAGTAA AGGACCAAGA CCTCAGGGGT 3600
CCCCTATCCT CAGCCCGTGG GGAGGTGGGA ACCATACATC GATCCCTCGG TTTATATATA 3660
GCCTCATCGC TGTGGGGCTC CGAGGTTGCC CCCAAAATCT TGCTCACCTG GAGGACCCCT 3720
GGGTGTCCTC GCCCAGAGGG CGCTGCAGCC TCGCACGTAG AGAACTAACA TCGCCCTTCT 3780
CCAGGGCAGT GCCTCCGGAC TCCGGACCAG GACATAGTAG CGAGTGCACC TGGGTCTCCG 3840
TCAGCTACGC ATCAAGGAAG GTGCGACGCG GGAATTACAG ATTGCCGGCA CTCACCAGTG 3900
CTCAGGGGAG GAAGGTGGGA CTCAGACCTG CAAGAGCTGA AGAGTGGGGT GGGCTTCGAT 3960
CCTAGGAGGC GTGGAAGGGG GTGTGGCTGG ATCCCTGGGG GGTGGGGCGC TCCAAGGGGC 4020
GGGGCAACCC AGGGGGCGGG GCCCGAGGGG TGGAGATTGG GACTACCCAG GCCCGCGGAG 4080

CTGGGGGTGG GCGGCTAGTT TTGCAACTGC AGAGCCTTTG GGTTTATTAT AAGGCGGAGC 4140

TCCGCGGGAG AGGTGCGGGC CAGAGGAGAC AGAGCCTAAC GCAGAGGAAG GGACCTGGCA 4200

GTCGGGAGCT GACTCCAGCC GGCGAAACCT ACAGCCCTCG CTCGAGAGAG CAGCGAGCTG 4260

GGCAGGAGCC TGGGACAGCA AAGCGCAGAG CAATCAGCAG AGCCGGCCCG GAGCTCCGTG 4320

CAACCGGCAA CTCGGATCCA CGAAGCCCAC CGAGCTCCCG CCGCCGGTCT GGGCAGC 4377

ATG AGA CGC GCG GCG CTC TGG CTC TGG CTC TGC GCG CTG GCG CTG CGC 4425
Met Arg Arg Ala Ala Leu Trp Leu Trp Leu Cys Ala Leu Ala Leu Arg
1               5                   10                  15

CTG CAG CCT GCC CTC CCG GTGAGTGTGG CCCGGGGCAG GGCTGGGAGG 4473
Leu Gln Pro Ala Leu Pro
            20

CGGCGGGAAG CCGGGACTCG CCACTCGCCG ATGCCATGCA GGCGGCAGCA CGTGGAGGGG 4533

GAGGGGAGCG GGGACTTCTT CCCGCGCTGC CTGGCGGATC CTGGGATGGT GAGCCCTTTA 4593

ATGAGGACTC CTGTCCCAAT TCCTCTACGG TCCGTGGATG CCAGGAGGCT ATCCCAGCTC 4653

GTGGTCCGGG CGTCCTGCAG AGTGGAACCT CCATTGGTTC CCCGCTCCCA ATTAAGTAAA 4713

ACGACTCCAC AGGGGTCTGA GTCGCCGGCC TTAGGCGCTC CGCCGGCCTT AGGCGCCGCT 4773

TGGAGTTGCT CTCTCCCGTT GCTGTCTTGC TGGCCATCTC AGCGGCCTGG CCTCCGCCAG 4833

TGTCCCGGAG GATGCAGTGG CCATGGCCAA ACGCCTTTTC CATAGACCCT AATTCAAACC 4893

AGACTGCAGG CTGCACCCCC AGCGCCGCGG AGTCCGGGCG CTCGGCCCTT TGCACCGGGG 4953

CAAGTTTGGG CACAGCAGAG CCGGCGCGGG AACAGGGGGA AGCTGACGTT CGGGGTGGCG 5013

GGAGGGACGG GATTAAGGCT GTTTGTGGGA CACAAGAGGG TGGCTCAGGG ACTTCGGTTT 5073

TTCTCTGGCT GCCCCAGGTG AGCCGGGCCG AGCTGGCAGC GGGAGGTTCC GGGAAGTTGG 5133

CTTCAGAACG CTGAAGACCC TAAGAACCCA ACTTTGGGGT CGCTGAAGTT GTGCTGCCCC 5193

CGGAGGGCCT CCTCCGCATG GCCCGCGCGG GGGACCCTCC CCGCGAGTGG ACCCCGGTAC 5253

GGCTCTTCCC CTCCCCCGAC TCGGCTTTGT GCTGAAGCCG CGCGTAGGGA AGGCGGGTCC 5313

CTTGGCCCGC CCAGTAGGGC CGCGGGGAAA GAGGGACGAA CGTGGAGCTG GCGACTGGTG 5373

GGGGAAGCTT CTGGGTAGGA TGCAGCCATC CACCTTTGGT GGGGTCGGTC TCTCTAATCA 5433

GCGGCTTGGC GACAAAGAGC TTGGTCGAGG GTACCCCAGA AAGTGCTCTC CCGCCCCAAG 5493

CCGCCGTCGC TAGCCCGCCT TCCCAACGGG CGCTTTGTTC TCGGCCCCTG TAACCCTTCC 5553

CTGGGAACCG CCCCGCAGCG CTGGTCCTTG ACGTGGCCGG GGTCCTGGGT CGCCGCCAGT 5613

GTCAGCGCTG CCCTCCGGTG TCCACGCCCC TAGCCCCCGC ACCCGCTGTG AAGTCCCGGG 5673

TGTCCTTTCC ACTGGCGCTT TGCCCAACCC CTGGAAGGCA GAGGCGAGGT GCGGAGCCTC 5733
AGGCTTTATC CTCCCGGAAG TGGCAGTCTC CCACCGCCAC ATCTGGTCTG CTTAACTTCG 5793
ATAGTCCTGG CAAAGGCAGA CACGTGCACA GGGAAGGAGA GTTGAGCGCT GGTAGATACC 5853
AAGGTCGTGT ACAAATAAAG TGGCACACGA CACGTCCCCA GTCACTGTTA ATGCATTGCC 5913
TTCGCTCCTT CCCAGGTGGC TGGTGCTCTC CATCACTCTG GAGCCCAAGA GAGGGCCTCC 5973
ATAATTGTAT TGCCCATGAG TTGGGGTTGT GTGGGGGCGC CAAATCAGGG TTCTCTGGGA 6033
GGGCTATGAA TTCCGAACTG AGTCTCCTGT GCACTCCTGG CTTTAAGGTT CAAGAAATTG 6093
TTTGAGGGTT GTGGTTTTTG TGGGACTCAG ATTATGCCTG GAATCATAGT TACCACTGTG 6153
GAGAAGAAAG TGGAGCTACT TAGCATGCCT CCCCGGCCCG CCTGGCATTA CCTCCGGCTC 6213
TGTTCTCTAG GCCCAACGTG AGGCCTCACT GGGGCAGTAC AGATGCAGTA CTGAATTTCT 6273
TTCCAGCCAG GATCTGGAGA GGTGGTGTTC TCTTCCCTGG TGTCTTTAGA GAGGCAGATA 6333
TTCCTGTGAC CTAAGCCCCT CAAGCACCCA TTAATAATGC TGAGTAGACA ACTAGAGGTG 6393
GCGTTTTCCG GAACTTCCTG TGTGCTGGCC TGGGAGGTTG AACCCTCTAG GAAACAGGTC 6453
TAGGAAGTAG AATTATCTCA ATGGAAGGCT TCCTGGAGGA AGAAGATGAG CTGAGCCCCC 6513
AGGTCACTGT CTGAGCTTTA GGATCAGACT CCCACTTGGA GGCAAGAGTG TTCGTTTTAC 6573
TTTTTTTTTT TAAGTTTAGT TTATTTTCTC TCTAACAGAA AACAAACAAA CAAACAAAAA 6633
AAAACCCCAC ATTGTTTAAA AGTGGGTGCA TAAGAGTGAG GACATATTCA GAGCTTCCCC 6693
TTTTCCTGAA AAATGAAGGC AGCTGGGATT TACTTAAAAT GAGAGCACAT ATCACAATTG 6753
CCAGAGAGCT GGTCCCTTTC TCAGGGCTCC CTAAGCTCCT GTGGGAAGCA GGTCAGACAG 6813
CCCTGGGGAC CAGAGAGATA GGGAGTGCTT TTGGGTGCCT GCCTTTGAAT GGGGAAGGGG 6873
GGGGGAGCTG CTGGGATCAG AGGCTGCTAG CAACTACTCC CCAGAGACTG AAGCAGGTTT 6933
GTCCCTCAGT GTCCTGTGGT CTTCTGTTTC TCCTATATAG AATAGGAGAA ATGGTTATTT 6993
GCTCTGGAAT AGTGACTTGC TATTTGTTCC CTTTCTTTCC TCTCCCTTAC TGTAATCATT 7053
TGGACTAGTA GAGACACTTT CCCCAGGTCT GGCAGAATGG GAGGGAGTGG GGGAGGCCTG 7113
TGCTTGCATG ATGTCACTGC TGGCTTCAGC TCTCCAGGGA GGGTGGAGTT GGTTGTAACC 7173
TACCTGTGGC TCTTGATGGG CCACAATAAA ACCTCATTAA CACACATTGG TAGGGAGAAG 7233
GGACTGGAAA GAATGATGGG AAAGATTGAT GTTTTTCCTT TTTTTTTTTT TTTTTTTTTG 7293
GCAGTACTTT CTAGATCTCC CCTCCCCCTT GCTGCAGCAA AATTTTGGAT TCCTGAAGTC 7353
CTTTGAGAAT GTATAATGGT AGCCAGACTT TTTTTTTTTC AGTCAGCTCA AAATTGCCTC 7413

CTTATAAAGT ATCCTTGGTT GTTTTTTGTT GTTGTTGTTG TTGTTGTTTT GTTTTGTTTT 7473
AAGACAAGGT TTCTCTGTAT AGTCCTGGCT GCCCTGGAAC TCAATATGTA GACCAGGCTG 7533
GCCTCAAACT CAAAGAAATC CACCTACTTC TAACTTTCAG TGCTGGGCCT AAAGGTGTAG 7593
GCCACCAAAA GTGCTCAACT TTTACAAAGC AGTCTTACTT TGAGCAGGAT TCTGAAACCC 7653
TTATTTCCTT TCTGTTATCT TCAACAATAC ACTGCTAGGT GTATTTAGTC CCTCATGATG 7713
CTGGGCCTCC TCAAGTGGCG CCAGGTCAAG CAGTCTCCTG GTTTTTGGTG GCTCTGAAGA 7773
AGACTGTGTC CCAGTGACTG GCAGTTTGAA TTCGGAGCTT CTCTTTTCCT TCTCAGTCTT 7833
TGGCAGGCAG AGTGACACTG GTGTGCCCAA GCCTGGAGCT TCTCTGTTTA ATTCTAGTTT 7893
ATTTTCTTTA TCAGACTGAA AAACAAATCA GGTTGGTTAT AATTCTTATA AACACGAAGG 7953
TCTCACCTTT GCGTACGTCT CCGGCTGTGT GGGTCTGATG TCCCTCGGGA ATCTCTGTTG 8013
AGGCTGCTGC AGTGTGTGTG CGTGTAGAAA GGGCAAGGTA GAATGGACAG AAGCGTGCTG 8073
CCCACCCCAC TGTCCTGTTC CTAAATGATG AAGCACTGGC CCGGTGAAGA GCCTAGAGAA 8133
CTCCCTCGGT GGGAGATGCA CACAATGCCA GGAAGCACAC AGGAGCTTGA GTTCCAGCTT 8193
GGCAGTGTCT TCTCTTTGGT GACTTTATCA GCTCCAGCTG CCCTGGACTA ACAAACAAGG 8253
CTAGCTCACT CTCAGTATTG ATAATCGAAG GTCCTTGGTT CTGTTTGAGA CTGATCCTCA 8313
CTCGGTAGCC TTGAACTCTT AGCAATTCTC CTGTCTCAAC TTTCAAAGAG CTGAAATTAC 8373
AGACTCGAGC CACCATATGC GACTGAAACC TTGTTCCTAA TCCTTGACTG TGAACGACTC 8433
TTGGGTTTGG TTCTTTCTCC ATTTCTTTAG TGTATGTTTT AGTTCGCGTC CTACATAATC 8493
TATTGCCCAT ACTTAGAAAC AACAGGTTAG AGACAGCATT GGGTCCAGCA GAGCCTCACA 8553
CTGAAGCTCA GTCCTGCCAC TGATTTACCG TGTCAGCTCA AGTGACTCAC TTCCAACTCC 8613
TCTGCTCCCC ATCTGTAGAG TAGACATCAC CATACCTGCT CTTTCTGCCC ACATTCTGTC 8673
ATTAACATGT TCATTTCATA ACGATGGTGC AAAAGTGCTT TGTAAGTAAA GTGCTGGGGA 8733
AATGTTAGCT GTCGATAATG GTTAGGGTTA ACTTTTTATT GAGTGCCTGT TGTGTGTGGG 8793
GTTGGGTGGG GTTTTTTTAG AGGCTTGGTA GTTTTCTTAC TTCTTTCCTA CTTAGCTTTT 8853
CTTCCTAAGC CTTTATGGTA TGTATCATTG CCTGATTGTT TGAGTGTGTG CACTGAGGCA 8913
CGCCTGTGCA TGTTTGAGAG TATGCTTGTG CGTGCTCTCG TGCTCACATA TGTATGGTGT 8973
GAATACACTG TAGAGTGCAG GCCGGCACAC TGGGGCTGGC TGAATCCTGT GAGCCCTGCC 9033
TGGAGTTTGC AGATCTTCCT TGGACACTCC TGCTTGTGAG CATTTTGTGT GGAGTGACTG 9093
TTTAGCTGGC TGTAGCCTAC ATTGTGCCTT TGGGTAAACC CTGAGTATTG GGAAACACCC 9153

TGGGCTGTGG CTGTGTGTGC CCGACGGTTG CTTGGGTACA GCTAAGAACT CTTCATAGAA 9213
AGTTGAGCTC ACATGCTATT AGTATTAACT GAGTGCTAAG GAACCTGTCT TGGGTGGTAC 9273
CTGCTTGCCC TCTCATGCAG TTTATCTTGA GCTTGGCGAA CACACTTACA GATTTAGTAG 9333
AGCTTTTGTC AGCCCTGGGA GGTGGGTTTC GTGGCCACAA GTGGGTAGCT TGGAATCCAA 9393
GACTCCTGGC TTCTAGGTTG CATTCTCCTG TGGTTCTTTC CAAGGGAATG CTAGGGGAAC 9453
ATTTTGGACA TTAGATTATT TCTAGTCCCA AAGCACACAG AACATACTGT TTCCTAATTG 9513
CCTTTTTTTT GTTTTCCTCT CAATCTGGTT TTGAAGTGTT GGGTTTGAAA ATTGCCCCCT 9573
GAGAGCCTGC CCTAGTGTGT GCAGAGGGAA GATAGTGGAA CAGGAAGTCT GTAGAAAGTA 9633
TCTTCCTTTC CAGGACCTTG TGCCCCGGAG CAGAGTCAGC ATGGTGTCAT ATCGCTTTTG 9693
GCTATTCCAG AAGAGATGAG GTTTTAGGTG AGAATGAACC TTTTAGAACC TTCTAGAACC 9753
TTCTGTTGAG TATGACAGGA ATGCCCTGAA TAGGGTCCGA AGTGCATGGC CACTTGTTTG 9813
TCTTTTCCAT AAGCAAGCAG CTTCAGGTAC AGACAATAAG ACTAGGTTCT TGGAGTGAGA 9873
CCCTGCACTT GGTGCCATTT CAGCTCCAGA TGGACACTGG AGGTCCCTAC ACAGCAGGCT 9933
CTGGGATGGC TGGCTTTGCT ATGTACTGTT GCCTGCTCTA CAAGAGCTTC CCAGGTTACT 9993
AGCCTTTGTC GACGCTGGGC TCGCTGGCCA GGCTTGGGCA TTGGAGAAGG GACAACTTGC 10053
CACCTGGCAT AGGCTGTGTG TTTGGAGAGT CAGGAGGTCT GGTGAAGCCC GCAAGTGGAG 10113
GCAAGTTTAG TGGGACTTGA GGAGAGCTCA GTAGGAAATC TCTGGGCTAG TGACAGGCAG 10173
GTGTGGTGGT GGTGGCGAGG TGGCGGGTCT AGATCTCCTT TTAGAGATTT GCCTAGGGAT 10233
CGTCCCTGCT GACTCTGGAA CTCAGAGGCC TCCAGAGGTG TCTCCTCTGG GAGCCTCTCA 10293
AGGGTCTCCC ATCTCCTACT GTTTATGGCT TTGTGGGCTA CCTAATTACA TAGAGAAGAT 10353
ATGTTCCTCT GCCTCCAGCC CTGGAAAGTT CTGCCCAGTG ACTCACCTGA GCCTGCAGCC 10413
ATGTGTGTAC ACAGGCGCTC TCAGGGGCTT CTGTCCTGCT GGCTTCAGCC TTTCTAGCCC 10473
CTGGTGTTCT CGGCAGTGGT AGCATCTGGG AAACCGGGTC ACCTCTTATT TGCAGCTCCC 10533
TCCCTTTCTT GGTGTCTTCC CCCTTTTTAA CTACTGGTCT GATGGCCTTA GACTCATGCT 10593
GAAATTCTCC TTTCTTTTGT CCTAGCCTTG TCTCTGACTT CTTGTGATCC TCTGGGCCTG 10653
TGAAATCCGC TCAGGGGCCT CCATTTCTAA CAGTCACACA CTGGTGGAGA GACCGAGTCC 10713
TGGGATGGTG AAGCTAACCC TGCTGGGCTT CTCAAGCTTC ATTTGGTTTC TCTTTATTCC 10773
TTCTGGAGGT ACTGCCTGCC CCAGGGGAGT CTCAGACTAG ACCACTCTGG AGTTGGAGGT 10833
GGGGCAGGTT TTCAGATCAG TGCCCTTGGC ATTCGTTGTG GGAATGGGGT GGATGGGGCC 10893

TCTGGGCAAG GTCAGGCTGG GGGTGGAGGC CAGGTGATGT TCTCCGCACC CACACCCAGG 10953
CAGCCTGGCA CCCTCCCCAA GGTCCGCTCA TCAGCAGGAA TGAAAGCAGT GCCGGGCAGG 11013
TTGGGGCAGT GGGCAGGTGG GCGTGTTTAT CGCTGTGCTC ATCAGCTGAG TCACGATGCC 11073
AGGCCCCACA AGTCCTCCCT GGAGGCTCAC CCCACCCACC TTGACCCACC AGCACCCACT 11133
AGCAGGAGGT AGGGCAGGGC AGTGAGACAA GACCAGCCTG GGGGTCTGAG AGGCAAAGGG 11193
GAGTTGTTCA TGACCTGGCT GTGCATGGGG ACTTGTGGGT GTCTCAGATA TCTCTGCTGT 11253
CCAGGAGGAA GCTGTCTTAA GTGCCAACCT GCCTAGAGCC CCTGCTGGGT GCAGGAAATG 11313
CACAAGGGAG AGTGCCCATC CATGGAATAG GCCCATGGAG CTAGACCAGT GACAGTGACA 11373
GTGAAGTCAG CCCCCACCTG TGTCTTCCGA GCCAGCTGGA GGGTTTTTAT CTCAGATTCT 11433
GCGAAACCAT AGAATCTAGT CAGGAGCCTA GACTGCAAAG CAGGCTTCGT TGATGCTTTA 11493
ACTTGCAGGC TTCCTGGGTA TGAGGGATAC TTAGAAAGGT CCCGCAGGTA GGGAGGGCAT 11553
CAGGAAGTAG AAGAGGGCCA GGCACTTCTA TCTCCTGCAT TGCCCCCTTC TCCCATCTCC 11613
AAGGATGGTA AAAAGAACCC TTCCAGTACA CTGACAGAGA GGAAAACCCT TCATCTCACC 11673
CCATTTGGAT CTGTCGTATC AGCATGTGCT GGCCCTGCTT CCATACCAGA GGTGGCTAGA 11733
GATGTTCCCT GGGAATTCAC TGGTTGGGGA CTTGAGTGTA TCAGAGGGGC ACAAAGTAAC 11793
ATTAACTCTG GTATCCTCTG CAGCAAATCG GAGATCCCCT CTCCTAGGCG AGTTCTCAGT 11853
GGATATGGAG GTCAGGTTTG GGCTTGTAGG GCCCCAGCAA GAGTCGTTGA TGTCACTCCA 11913
GCTTCTCCCG AGGAAGATGA GGGTGCTGTG TTGGGATCAC ATCTCTCCCT GAATGGCATG 11973
TTGGGGAGGG ATGGAGCCCT TGCTTCTGAC CCCTAAGCTT GGTCTTTAGG TGGCCACAGT 12033
CTCTGGGTTC TGTCCTACCT CCCTGCCCTT GTGTGCTTCA AAGGCATGCT AAAGGGACTC 12093
TCGGCCATTC CGAATGGCAC AGTGTTCCTT CTGTTCTCCC ACCCCCAGAA GGAGGCAGGC 12153
CTGGATTGTA GATTCCTAGA AGTAAGTGGC CCTGAGCATG CTGTTGATGA ACCTGGAACC 12213
AGGCAGGCTG GGCATCCTAG GACCTGTCTT TCCATAGAAG TCTGAATCAG TCTACCTTTG 12273
GGACTGAGTA AGGGGCTCCT CACATATCAG CTGGCTAGTC CATCTTGGCT GATCTAAACC 12333
ACATTAGGCT GAAGAGAAGC ATGGTGTACA GTCTGGTCCA CCCGAACCAC ATACTGGCTT 12393
TATCAGTTCT CGTATAATTT TGCAGGTAAC TTTTTAGCTC TAAGCCTGTC TCCTCATCTG 12453
TGAAATCGGG TCCCTCATAT CCTGCCTAGA AGGGCTTTTG AAAAGATTAA TGAAGTAGTA 12513
TGCCGAGTGG TTGGGGTTCT CTCCTTGACT GGAGCAAGTC TCTAGGAGTA CTAAGGATAG 12573
CCTGCTGTGT GCAGCACCCC CAGGGACTGT GCCTGAGTAG GAGGGTACAG AGTCTTCATG 12633

TGAATGGCCC TTCTGGTCTT GCCCCGAAGT TAGTGTTGAT GTCATAGAGT CTACAAACAT 12693

GCCTTTTGTC CTTCCTCAGA AGTCCAAGCC TTTCCTGGCA GACCAGACAT TCATCTCCAC 12753

TGAGCCTCTA TGTGAGACTG GCTCCTGGCC TGAGCTGTGT GGGCTGAGCT GGCGAATGGG 12813

AAAACTAGAC ACCTGGGCAC CTGGGTGGGG GCTCGGGACA GCAGTGTTTC AGTTGTAGGC 12873

ACTGTGCCCC TGCCTGGAGC TTCTGACTGA AGGTTACCCT GAGAGGAAGC AGGTTCCCTA 12933

TAGACACTAA CATAGCTGGG TCAGAGTGCA AGGTGGGTGT GCCCCTGCCC TGACCCATTC 12993

AGTGCAAAGG CTGCTCTTCT GGGAGTGAGA GCTCTGACAG GACTGTGATG GCCGAGGGGT 13053

CTCAGAGCAA ACCTGCCTGG CCTCTCCCCA CTCTGATGGA TATGTGCTCT TAAACAAGTG 13113

ACTGTCCACT TTGCCTCAAT TTCAACATCT GTAAGATAGA TAGGGCGTTA TGGTCTGAAA 13173

ATGGTTTTAA AGATTAGTTA GCTAATACAG GGAAAGTGCT CTGACAGGTA CCTGGCACCT 13233

TACTCAACAA GTGGCTGGAG TGCCTGATTT CCTAAGGTCT CGACCTGTCC CTATGCTTCA 13293

AGTGCCCCTA CAGCCTTGGT CAGGCCCTTA GGTTCTCCCA CCCACCGCTG GCCCCAGGAC 13353

CTAGACTGCT GGACCCTGAC CCCATTTTTC CTTTAAGCCA CCTCTGCGTC AACTCTAAAA 13413

GGCGGTGGAG TTGTTTATCT AGGCTGTGAG GTGTCAGAGA AAGGACCTGG GCCGCTTTGT 13473

TCCTGTGTGG GCTGGGGCCA CTCCAGGAAC TGAGAAACCC ACCCACCTTT TCAAAAACAG 13533

CCTCTTCTCA GAGTCTGGCA CCTCAGCTAG CCACCATGCT GTGGGACCAC TCCCAGCATG 13593

CTCTGCCTTT GGTTTGTTTC CCAGGGGCCT CAGTGCCTTT TAAAGATGCA CAGGCATCTT 13653

TAGTTCAAGG GGAAAGAGGA AATGAAGTGT ATTTGCTGGT GGTGGTATTC CTGTCACTTG 13713

CATTCTCACA GAGGCTAAAG AAATTTGCTC TTTGTATCTT CTAGTCTCTT CTTTATGATC 13773

TTTTCCCATC TGTTGTATCC CAACTGCAGG GCCCCAGTTC TAGAATTAGC CCCTCCCCCA 13833

TAGGAAGCCG ACTTATGCTA TAATGTGAAT GACAAGTATC CTTTAGCCCT TCCCACAGGC 13893

ATTTTAATTT TCAAAAGGGC ATTGCACAAC CGCAGAGACA CTAAGAAGAG AGGTTTGGTG 13953

ATCAGAGTTA CAGCCCCAGC CTCCCAGCTG GTGGCCCGGC TGGTGCAGGT GTGTCGAAAG 14013

CAGTAGTTTC TGCTTCAGTG AAACTTGAGG ATCCTTTATT TAGCCAGTTC AGGGGCGGAA 14073

TGGCCATGCG AAGTCTATGT GTCACAGGTG TCAGGCCCCC ATATCCTGCT GAGTCTAGAA 14133

TCAGCTACGT AGCAGTTTTG GGGGTATTGC CAGACTGGGA GTTTACATCC CAGAAGCGAG 14193

AATGGTGGGG TTCCTATACT GCTCCAGACA GGATCTTTCC CCCAAGTTTG TCAGCCACCT 14253

CTCTTCAAGT CCCTTGGCTC TGACCAGCAA GACGTATCCA AAAGAAACTG AGGAGGCCCT 14313

TCACTTCTTT TTAGGATAGT GTGGGGCCAG CATGGTGGGG GTTGGGAATG GCTTTCTGTC 14373

TCTTCCATCA TCACAGGCTA CTTCCCAGAG ACACTTTGAT TCTGGGCATC TCCAGCAGTC 14433
ACCTGGCCCA CAATGCTTTG CTGCCCTTTG CTTCAGCCAC TGTATCTGGT TGTCCCTTGA 14493
AGGTGAGCCA GAGCTCCTAG GCAGAGAGCA TGTGCTATAC AAAGCCGTAG GCTGGGCCCT 14553
GGGAACCTTC TTGCTGTCAT CCTCCTGTCA AACCCCTATG GTATGGTAGC CCACATAAGG 14613
CTTGTGCAAA AAACAGGCCA AAACATAAGT TATCTTTTCA CTCTATCGGG TCTTCTCATT 14673
TTCCCATGGT ACGTTCGGCT GGCCAGGCCC AAAAGATTTG AAGAGAGGTG GCTGGCAAGT 14733
CTAGGGGAAT AGGTCTATCT GGTTCCCTCC AGGAGCAGTG CCTAGTGAGA GGCTGGGCTG 14793
GGCAGGGCAG GGCCCCTTGC TCCACATTGC CTGAAGTCCC GCCCTGCCCG TCCTGGCTGG 14853
GATCTGGCAG GTCTTCCAGC TCCACACCCG GCTCTCAGCT GAGCCTGCTC AGAGACTAGT 14913
CCTGGCATGT GGGTTGCAGG GCTGGTTCCA GCTCCACCAG GAGGTATGGG CGTCTGGGTA 14973
CTCATGGGAC ATTGACCTGT AGTGGGTATG GAGAGTGGAG GAATGGTACA GGCAGGTGTG 15033
CTGGTGCTGA CGGACTTGAC TCCGGCATTG ACCTTGGCTT GCAGTCTGGT GTTAAACTAA 15093
CAGGGAATGC TGACAAAAAA GACAGTTATT AAAACCAAGA CAGGATACTG CTTTCCCACT 15153
CAGCCCATTC CCAAGAATCC CCAAGACGTA CAGGAAATGT GCAACAGCAG TGGGAATTGC 15213
TGAGTTGGGG GATGTGGGTG AGCTGTGTGC TCCCAGGGAA TTTTGGGAAA TTCCCCTCCG 15273
TTGAAATGCT GTCAGGGTCT GAGCCTTGGA GGTGTTTTTG GGGTGCTGTG CTCCCCAGCT 15333
AAGCAGCTAA CAGTCCTCTT TACCTGCCTT GTCCTCACCT TGCCCCACCC TGGGTTGGGC 15393
CTCTCGTTCA CTCCCTGCTG GGTCACCAGT ACTTCAGTGC AGGTCTCAGC TTGATTCTTG 15453
GTGGAGAGAG AGAAAGTTGA TAAATCAGGG TGCCTGTCAG CCGGAAATTT GGGTGTGTCC 15513
TGAAGGCACC AATGGGGGCC CTCCCTTCTG GAGGTGGCTT TAGGAAGGGG TTTCTGGGTC 15573
TTGAGGCCTC CTTACAGTTT CTTAGCTCCA TGGGAGAGAA GTGAGGAGTT GGGTATCGTC 15633
ACCCCAGCAT GAATCTCTGG TCACCTCTCA GCATGCACTG TCCAGCCTGA TCTTTGAGTG 15693
CCATAAAAGA ACAGAATTAT CCTCTCAGAG CACTTCATTT CCCGCCAGCA CAGTGGGTAC 15753
AGAGACAAGC TGCCCAGACT CCCAGCGAGG GACTAGTTGA GCCCCAGCAT GGGACTAGTT 15813
GAGCTAGACC TGATACAGTC CCAGAGAGCC TCGTTGAGGA AGCTTTGGGA AAATTCACCC 15873
AGCATTTCAG CCAGGACTGG AGGAAAAGGT GATTATGGGA AAGAGAGCAG TCAAGACCCC 15933
AGGCTGTAGG ACACAGGATA CAAACTGAGA GCTACCGGAT AGGAGTAGGT TTTAGTCACA 15993
ATCTCTCCTG TCCGCCCTAC CCTCCAGGAG ACATTGCACC TTGTAGAACA GCTGCCCCGG 16053
AGTCCACCTT TGGGCCCCCC TGGGTAGCTC AGTAGTGTCA GCATCCTCTC ATTGACATCA 16113

GTCAGGTTAC ACAGTGGGGC AGCTAATGTG AAGGCGCTAG GCTGGGAAGC CAGCTACTTG 16173
GGAAAACTAG GTTGTTCCTG GTAGGCCCTA GCAGGAAGGC AGTTCCTCCT TTTCTTGGTG 16233
GCTTTAGGGG TCTTTGGAAG CTTTGAATGT TCCCTCAGCT CGTTGGTGAA GCAGGCCCTC 16293
CTGGTACTGT GGTGTTTGTC TTCGAAGAGT GAAGGCATTG GAAGTAAAGA CTGATGGGGC 16353
GCCTTCCCAG GATGCTTTGC TTCTTGCGCT GGCTTACAGA GCTCTCTTGC TACCTAGTGC 16413
CTTGACTTTG AACACCAGAT TCAGTCAGGG AACAGGAGTA GAGGTCTTGC CTTGCTGAGC 16473
CCCTGCGCAC TGCAGGAAAA GACTCCTCTG AGTGGAGCCT TTCCTCCTCA GGTGACTGCT 16533
TTCAAAGTAC AGCAGCCTCT GAGGGGGAAG TGTCATTTGA CATTGTGGTA GTTCTTGGGG 16593
TCCCTGGATA CAGATGTCAT GCCCAGATCA TAGGTCTGTT TGTACAGAGG GAGGCGAGTT 16653
CTGTAGCTCA GAGTCCTCAG TACCCCAGAG TTGTGGCTCT AGGGGTGAGA GGAGAAGACT 16713
ACAGCCCTTC AATCACAGGT CTGACCTGTG GGTAGGGGTA GATCTCTTGC ATACTATGAA 16773
CCTGTTTGAA ACCCCTGGGT ATTTGCTGTG GAATAGAGTC TTGGTTGGGT AAGAATGGTG 16833
GATGTTTATC TTGGTGTGAC TCTCGGGTGG GGGTGGGGGA TATGTCCCTG TCTTTCCCAA 16893
TGTAGTATGC TGAGTGGACA GAGACCGTGT GACTGAAGCC TGGGCTCCTG GAACAGGTGT 16953
GTGTTGGTGG GGGGTGGGGC GCAACTATCT GGGATCCAGA CTGCTTGGGA ATGGCTGTGA 17013
CCCAGCTCCT TTGATAACAG CAGCTCTTTG TCACTGGATG TTGTGACTAA TGGGACTTGT 17073
TGATTCAGTT ACTCGGCTCC CACCCACAGA CGCCGGGGCT TCTGTTGTGG CACCAGGCAG 17133
CTGCAGACGG CCCACAAGTT TGCCTCGCTT TCCCACTCCA CGAAGGTAAG TTCCCAGCAC 17193
TGCCCAAATT AGAGACTTGT GAGTGGTCCC CTCATACCCC ACTCCCTGAG GCTTCTCCTG 17253
GAAGGCCTGG AATGGGGCAC TGGGTGTGTA CGTGCTGTGG TTTCTGTTAG GGTCAAGACC 17313
AGGCTGTTTC TTACCTGGCT CGTACCTCCA AGTTTCCAGG TGATGAGTCC TGATTTTTGA 17373
AGTGAAGGAA TCCATTTAAT ATCAAAATTC TGTGACCTTA AATTTTTTTC TTTTATTATG 17433
TGTCATTTCA TATGTACGCA TATTTTTTTG TCTGTGTGTG GACATGCTTG TGGCGATCAG 17493
AGGACACTTC AGAAAGTCAG TTCTCTCCTG CCGTGTGGGT CCTGGGGAAT CAAATCCAAG 17553
TTGTCAGGCT TTATCCTGAA AATAAAAAGT AGACAGCCCT TGGGATCCAA AGCTTCTTAG 17613
GGCTGTGTGT CTTAGACACC ACCAGTGTTG CACAGCTGGT AACATGACAG TGTCCTGGAG 17673
TGCTGATTGG AAGCCACAGG CCTCTGTGCA GGGCGGTAGA CTTCCAGGGT ACGGGGCAGG 17733
TGGGCGTTCT CTACAAAAAC CTTGTAATCG CGGACGTCTT GGAGATGCCC CCTAGGTATC 17793
ATGATTTTGG TGTGTGACAC AGCTGAACTG TCTTCATACT CAGGATATCA TGAAGTGCTG 17853

GGGTGCAGAC CACTCTCAGC CTCAGGCAGC CAGGACCCGG GGCTCCATCA GATTGCGGTG 17913
ACTACCACAG AGGGTGGCCT TCCTTCCGGT CAGTGTGGGT GTGGGAGCTG GCAGGAAGTG 17973
GCTCCAGGCT TCCTTTAAGC ATCCTCTGCC CACAGCCCCA AACATGTTCT TTGGCAATGG 18033
CTTGCAACTA GAGGTGAACT CTCTCCTGTA CTATGTCCTG ACCCACGCTG CTGCATCTAT 18093
TATACCTTTC ACACGCGTGA TGGGTACCCA GCGGGGCTGC TAGGCAGGGT TAAGCACTCA 18153
TCTTGTTTCC TGGTGCTGAA GCTGTGGTAA AGAAACTGAG GCCATTTTCC CTTGAGAGAG 18213
ATGGTCTCAG CCAGGTCTTT CTCGGCCTGG GGAGCCCGGA AGAAAGGATG TACTACAGTG 18273
AGTGGACACT TGTTGGCTGA TGGCCTTGGT AGGTCCTTCA CCCTGGGAAG TGCTGTTTCT 18333
TATCTGTTAG AGATGCTGAC CTCAGCAGGA CTGGAGGAAC TGCATGGGAG GTGTAGGAAT 18393
GAAAGTGAGT GGGGAAAATT ATCTCCAGCC CTAGGGAAGT CTGAGGCCTG TGTCCCCTTT 18453
GTCCTGGACT GGGCCCCTGC CTTGGGTGTC TGTCCAGGGT CTTTGCTCTA CAGCCCCAGC 18513
GGATGCCCAA AGTAGACGAG TCAACTGGTC CTTTCTTTCA CCCTGTGTCC ACTTCTCATG 18573
TATCTACCTT CATAATCCTT CTAGGTAAAA CAAGCCTCTA ACTTTGGGTT TTCAAATCAG 18633
CCAGCTTCCA GGCTCGATAG TACGAACCAT GAAAATCTTT CTTACCATGA GGTTGTTTTC 18693
TAGTGTGTGT GTGTGTGTGT GTGTGTGTGT GTGTGTGTGT GTGTGTGTAC GTACACATAT 18753
GTACCTCTAT CAGTGTGCTG TGCGTGTACC ACAGCAGACT CGTGAGGAGG TCAGGCAAAC 18813
TTTATAAAAA TCTTTTTTTT TTGCTTCACT TGAGTCCCAG GGTCACACAG TGGCAAGTGC 18873
TGAGCTCTGT TCTCTGTTCT TGATTTGTTT TGTGAGCAGC TGATGTTCTT AAGGCTTGCG 18933
GAGGGGAAAG GTAGGGCTGG CTTGCTTCTT CCCCGAGTGG CGGTCAATCC CTAGACATCT 18993
CTAAGCCGTG GCCACACGTC CTGGAAGGAC CCAGGTCAGA AGTGATACTG AGATGGCCCT 19053
GTGAGCCCTC TCGAACACAC AGGGTTGTAA ATAGTACCTG ATTGTTACAT TGGAGACTCG 19113
TCAGCTGGGT GGAGTCCTGG TTCAGAGGGA GTTATTCCTC CCCCCACATT TCTTCTCTTC 19173
TGGGGCTGAA GTCTCTTCCT TCCTTACCTG TGATGCTGTC ATGATAGGTC CCAGCTGAGA 19233
GTGGAGGCGG GGCAGTCAGG GAGCTGCTTC TCTTTGCTTA GCAGGGGTTG GAGACTTGGG 19293
GTGTAGGGGT TGGCTCCCCC TTTCCCTGCC CTGGACCTGG TTTCTGGTTT CAGCAGAGAT 19353
TCGTTCTAGA AACTTGTTGC GTAAACAAGA TCACAAAGCG ATAAGCTTGA GCAAAACCCA 19413
GGGGAACAAA TTGCTTCCCT GTGAAGACCC AATCTTAGCT CTTAGAGAAG CCCTCCCTTT 19473
TGGAAATTGC TGACTTTCAG GGCTTCTCTG TGGAGGAAAG AGGCTAGCCG CCGTATGTTT 19533
GCCTGGATTC CAATAAATCT TTGCGGCCTT GGCTACCCCT TGTTGAACAA GGTCTGCACT 19593

CCTAATGCGT GCCTCAGGTG GTCTGAGACC TCTACCCCAT CTCCAGCTTT TCCTTCCTAT 19653
GGAGGGAGTC AGTGGGTTAG GAGAGAATGG AGTTGAGTCC TGGAATGAGG AGGAAGCTAT 19713
GAACTCGGGG CCTGTTCCTG TCTGGTGGGT GCTCTTCTCC GCCGCTGAAG GAGGCAGCCG 19773
CAGGGAAGAC TACCACAGGA ATCCGAGTAC CACCTGGAGC AGTGTATACA GGATGTGGGC 19833
TGATGTGTGG TAAGGGCATG ATGGGCTGAT GTGTGGTAAG GGCATGGGAT CTGATTGCTC 19893
TGTGGATGGG CCACAGGGAA ATTTTTGAGT GTCTACTGCA GTAGTTCTCA ACCTGTGGGT 19953
TGTGCGCCCC TTGGTGGGAG TTACATATTA GATATTTACA TTATGATTCA TAACTGTAGC 20013
AAAATTACAA TTGTGAAAGA ACCAAGAAAT CACCGCAGCA TGAGAACCTG TATTAAAGGG 20073
TCACGGTGTT AGGAGGGTTG AGAGCCACTC ATCCTCTGGG TCTAGGCCAT GGCGGGCTGT 20133
AACTGCTCTC TGGAGTTAAG CCACAGTGAA CCAGCTGTCC TTGCAGATGG ACTTGTGGAG 20193
GCTCCAAACC TTTGTCCCAG GGGAGAAGAG CTTGCTTTTG CTTTGTACTT TTAAAGGAAG 20253
TTCAGTGGTC TTCGGGCCTT GTGGCTGCTG TGTGTGGAAG TGCCCCTGTA CAATAAGCTG 20313
TATAGATCGT GTACAACTGC AGTTTTCCTC CGTGGGTCCA CCAACCACTC CTGACTCCAC 20373
GGATGAGTGA GGCCAGTAGG GCTGTGTGTG GGTCCCTAGG CCAAGCATCC TGGACCACGA 20433
TGAGCCTCAG CTAGACCACT CTGGATCTTT AGCAGAGGCT CCTAGAGAGC TGGCTGGCTT 20493
CCTCCTGCCT TCTTTTCTCT TAAAACTTCG TCTCAATCGG AAGCTCCTCT GTGCACGTGA 20553
CCTCCAGGCC TGGGGGTCGC CACAAATCCC CTCATCACAA GACGAGCAGC TCGCATGAGG 20613
GACACGACAC TTGTTACCTA CCAGGCTGTG GGGTTTTTGT TGGTTGGTTG TTTTGTTTTG 20673
TTTTGTTTTT TTACTTGTAC AGAAGTGTTG TGACATCAGA TGTCAGCTGT TAGTGCTGGC 20733
ACCATTTTAC AGGTAGGGAA CTGAGGCTGT AAGATGTGTA GTGACATCGC TAAGGCCACT 20793
CAGTTGGTGA GGCCTTACCA AGGTCAGGTC TTTGGAGCCT TTTGCTGAAC CATGTACTTC 20853
TATCTCTGTT TTGTTGAAAC AAAGTCTATA TGGCTCTGGC TAGCCTATAA CCCCATATGT 20913
AGACGAGGCT GACCTCGAAT ACACTGCAGT CTTTTATGTC TGCCTTCTGG GTGGCAGGAT 20973
TGAAGGCATG TGATTCCTCC TAACTGTACA CTTTAAAAAA AAAATCATTC TTTGTTCTGG 21033
TCTGTGCCAG GGCCTTGTAA GATGTTCTGT GCTGAGCTGG GCTATTTGGG TTAGTCTCAT 21093
TGCTGAGCAG GGCCCCTGTA TCTTCCTTCT CTGTCACTTG CTTACCTGGG TCTTCCTCCT 21153
GCACTAGCTA TCCTAGAACC AGTACTGAGA GCAACTATGG GCCCAACTCT GCCCCTTGCC 21213
CAGCCTGCTT AGCTGGGGGC GGTGTTCCAC TTCCCTGCCC AAGTCCTGTG GGACTGTGTT 21273
TGTACTCCAC CACCTTCAGT TCCTTGGAGC TGGAGCAGGC CAGGCGGCTG CATTCCTGCA 21333

GCTGCTGTTG CCAGGGAGAG CCCATCCCAT TCACTTCAGT CTCCTTAATG TAGAAGCCTT 21393

GTCGAATTAG CTTCCACTGT CCCCAACCCA AGAGTACCCT GTCCTTTCTT CACTAAGAAG 21453

GCCAGGATAC AGTCCTTCCT GTGGCTGATA AGACAGGCCT TGGGACAAGG CCTGGGACCA 21513

CACTGTGTGG GCAAAGCTGC TTCAGCACCG ATGGCTCCTC CATGCCAAGC TTGGCTCTGC 21573

TTCTCACAGT TGAGACTTCT GTGCGCACAC CCACTGTCTA GCTCAGCTGG ACACTGATTT 21633

TCTTTAAATG TATAGATTTT GGGGTGGGGT GTGCTGAAAG CTCCCACTGA TGCCCCAAGC 21693

CTGAGTCTCA GAGTATGATC AATTGATGGC TTTCATGGGT ATCACAGCTT CTGTTCCCAG 21753

GTCAGACTCC CTGACCAGTC AGAGCATCCT GGGGTTAGAC AATGTCCCCG TCACTTGTGC 21813

CTCCACCTGG CACCAGGCTA TGATGTTATG GCATTGAGGG TATGAGAAGG ACCAGGGGTT 21873

TCCCAGAGTT ACGCCCAGGC GCACAGGCAA TTGTTTCCTA CATGTGTGGC TGGAATGGTT 21933

GGGTGAGCCT TTTCAGCTGC CTACAATAGG AACCCAGGGA TACTGGGCAT TGACCAAGGC 21993

ATATCTCATA CCCTTTTCTT ATCTTTCTGC AG CAA ATT GTG GCT GTA AAT GTT 22046
Gln Ile Val Ala Val Asn Val
        25

CCT CCT GAA GAT CAG GAT GGC TCT GGG GAT GAC TCT GAC AAC TTC TCT 22094
Pro Pro Glu Asp Gln Asp Gly Ser Gly Asp Asp Ser Asp Asn Phe Ser
30                  35                  40                  45

GGC TCT GGC ACA GGTAAGACTG ACCCAGAACA CTGAGATGGC ATAGATCATG 22146
Gly Ser Gly Thr

GCTGGAGTGG TGAGCAGGCA GTCACCCAGC TTTTAGTGAA CCCCCTTCTT CTCCCATCCC 22206

ATCCTTAGCC ATTGGAGTCA GGACAGTGCC AAAAGGAAGA ATGGTATCCA GCTGCAAGCC 22266

ACTCAGCTAA GAGAAACTCT CAGAGAAATG AAGGGGTTCC ACCAGGCCAT GGGCAGCCAC 22326

TAGAGCCAAC CCTTGGAGGA GTTTGACTCC ACTGAGCCTT GGTGTGGTGT TTCCATCTGT 22386

GAGATGGGAA TACTTTGCCC AAGAGCCTGT TAGAAGCTGT AGGAAGCACA GAGTCGGCTA 22446

GGTATAGATT TGCTCTCACC TCCATCTCTC GATACCAGTT CTCTGCAGAG CTTGGGTTTG 22506

TGGGAGGGGT GGGGGGGTGA GGGGAGAAGG CTGTGAGCTG CAGCTAGCCA GAGGGGTCTC 22566

CCAGAAGAAT GGGGAGAGCT AAGAAGGAAA GTTGAGGTCA CAGTGGGAAG GAGACCAGAG 22626

CAAAGGGTTG GAAGGTAGGT AAAATGCAGC CGTGTATTCT TGGGAGCCTT AGGCCTTGGG 22686

CAAGAGGGTA GAAGAGGTGT TTGTCCTGGG CTGCAGTCCT GTATCAGCTC TGGTGTCTTG 22746

GCCCACGCTC ACAGCAGGAT CCCTTCCCAG ATTCCCGAGA ATTTCTCACA GTTCAGAGAG 22806

CACGCTACTT GTAGGCAGGT GAGGCTGCAA AGGACAGCTT TTCTGGCCTA ATTTTCAAAG 22866

TGAGTTCAGC CTTTGCTAGG TCACCTTTGG GGTCTCAGAA GGCTTCAGCT CCTGGTAGAG 22926

CATGAATCAC GTCAGGCGTG ATGCTGGAGA CCTCTCCTAC CCTGACACCC CAAACCCCCA 22986

CCTCTGACCC TGCA GGT GCT TTG CCA GAT ACT TTG TCA CGG CAG ACA CCT 23036
Gly Ala Leu Pro Asp Thr Leu Ser Arg Gln Thr Pro
50                  55                  60

TCC ACT TGG AAG GAC GTG TGG CTG TTG ACA GCC ACG CCC ACA GCT CCA 23084
Ser Thr Trp Lys Asp Val Trp Leu Leu Thr Ala Thr Pro Thr Ala Pro
            65                  70                  75

GAG CCC ACC AGC AGC AAC ACC GAG ACT GCT TTT ACC TCT GTC CTG CCA 23132
Glu Pro Thr Ser Ser Asn Thr Glu Thr Ala Phe Thr Ser Val Leu Pro
        80                  85                  90

GCC GGA GAG AAG CCC GAG GAG GGA GAG CCT GTG CTC CAT GTA GAA GCA 23180
Ala Gly Glu Lys Pro Glu Glu Gly Glu Pro Val Leu His Val Glu Ala
    95                  100                 105

GAG CCT GGC TTC ACT GCT CGG GAC AAG GAA AAG GAG GTC ACC ACC AGG 23228
Glu Pro Gly Phe Thr Ala Arg Asp Lys Glu Lys Glu Val Thr Thr Arg
110                 115                 120                 125

CCC AGG GAG ACC GTG CAG CTC CCC ATC ACC CAA CGG GCC TCA ACA GTC 23276
Pro Arg Glu Thr Val Gln Leu Pro Ile Thr Gln Arg Ala Ser Thr Val
                130                 135                 140

AGA GTC ACC ACA GCC CAG GCA GCT GTC ACA TCT CAT CCG CAC GGG GGC 23324
Arg Val Thr Thr Ala Gln Ala Ala Val Thr Ser His Pro His Gly Gly
            145                 150                 155

ATG CAA CCT GGC CTC CAT GAG ACC TCG GCT CCC ACA GCA CCT GGT CAA 23372
Met Gln Pro Gly Leu His Glu Thr Ser Ala Pro Thr Ala Pro Gly Gln
        160                 165                 170

CCT GAC CAT CAG CCT CCA CGT GTG GAG GGT GGC GGC ACT TCT GTC ATC 23420
Pro Asp His Gln Pro Pro Arg Val Glu Gly Gly Gly Thr Ser Val Ile
    175                 180                 185

AAA GAG GTT GTC GAG GAT GGA ACT GCC AAT CAG CTT CCC GCA GGA GAG 23468
Lys Glu Val Val Glu Asp Gly Thr Ala Asn Gln Leu Pro Ala Gly Glu
190                 195                 200                 205

GGC TCT GGA GAA CAA GTGAGTGGCT TTGCATTTCC TGGGTGGCCA CTAGTGCCTG 23523
Gly Ser Gly Glu Gln
                210

CACCTGGCCG CCTAATGTCC TCATTACAGT GACAGGTGAC AGGGTCCCAC CTTCCTCCTG 23583

CCCGAAACAG ACTGATTGCA AGATCAGGAG GTGGGCGACT CCTTAGATGT CATTCAGGAG 23643

CTTACAGCAG GGTGAATTTT CCGTCTTAGA CCTTCATGGG AATTTTCACA CAACAATGTG 23703

TACGTTGTGT CACTGGAGGC GGTATCTGTG TCTTGGCCTG CCAGGGTCCC AGGTGTGACT 23763

GACTGCATTC CTTGACAGAT GCTGGTATAG GTTGGCTACG TCTGATGGGG GTGGCAGGGG 23823

ATCCCATCAG GTATGGCACT GCTCAGGTTG CTGTTGTGTC AGTGGCTCCA GCTGACCTGA 23883

TCCCAACCTA CCCTTCTGTA G GAC TTC ACC TTT GAA ACA TCT GGG GAG AAC 23934
Asp Phe Thr Phe Glu Thr Ser Gly Glu Asn
                215                 220

ACA GCT GTG GCT GCC GTA GAG CCC GGC CTG CGG AAT CAG CCC CCG GTG 23982
Thr Ala Val Ala Ala Val Glu Pro Gly Leu Arg Asn Gln Pro Pro Val
                225                 230                 235

GAC GAA GGA GCC ACA GGT GCT TCT CAG AGC CTT TTG GAC AGG AAG GAA 24030
Asp Glu Gly Ala Thr Gly Ala Ser Gln Ser Leu Leu Asp Arg Lys Glu
            240                 245                 250

GTG CTG GGA GGTGAGTCTT CTTTCAGGTG GAGAGGAGGA GGCAGGTGGT 24079
Val Leu Gly
        255

GGCTCTGAGG TAGCCTGGGT TGCTGGGGTG AAGCATCTTT AGCAGCAGGG TGGGGAAGGA 24139

GGAGGGTCAA TTCTACTCCA GGCCACCTCC TAGGCTGTCC GTCTAGTCTG GGAGAGACTA 24199

CCACTGACCC CGTGGAGCTA CTGATCTGAG CCTGCCTCTG TTCACTCCCT A GGT GTC 24256
Gly Val

ATT GCC GGA GGC CTA GTG GGC CTC ATC TTT GCT GTG TGC CTG GTG GCT 24304
Ile Ala Gly Gly Leu Val Gly Leu Ile Phe Ala Val Cys Leu Val Ala
        260                 265                 270

TTC ATG CTG TAC CGG ATG AAG AAG AAG GAC GAA GGC AGC TAC TCC TTG 24352
Phe Met Leu Tyr Arg Met Lys Lys Lys Asp Glu Gly Ser Tyr Ser Leu
    275                 280                 285

GAG GAG CCC AAA CAA GCC AAT GGC GGT GCC TAC CAG AAA CCC ACC AAG 24400
Glu Glu Pro Lys Gln Ala Asn Gly Gly Ala Tyr Gln Lys Pro Thr Lys
290                 295                 300                 305

CAG GAG GAG TTC TAC GCC TGATGGGGAA ATAGTTCTTT CTCCCCCCAC 24448
Gln Glu Glu Phe Tyr Ala
                310

AGCCCCTGCC ACTCACTAGG CTCCCACTTG CCTCTTCTGT GAAAAACTTC AAGCCCTGGC 24508

CTCCCCACCA CTGGGTCATG TCCTCTGCAC CCAGGCCCTT CCAGCTGTTC CTGCCCGAGC 24568

GGTCCCAGGG TGTGCTGGGA ACTGATTCCC CTCCTTTGAC TTCTGCCTAG AAGCTTGGGT 24628

GCAAAGGGTT TCTTGCATCT GATCTTTCTA CCACAACCAC ACCTGTTGTC CACTCTTCTG 24688

ACTTGGTTTC TCCAAATGGG AGGAGACCCA GCTCTGGACA GAAAGGGGAC CCGACTCTTT 24748

GGACCTAGAT GGCCTATTGC GGCTGGAGGA TCCTGAGGAC AGGAGAGGGG CTTCGGCTGA 24808

CCAGCCATAG CACTTACCCA TAGAGACCGC TAGGTTGGCC GTGCTGTGGT GGGGGATGGA 24868

GGCCTGAGCT CCTTGGAATC CACTTTTCAT TGTGGGGAGG TCTACTTTAG ACAACTTGGT 24928

TTTGCACATA TTTTCTCTAA TTTCTCTGTT CAGAGCCCCA GCAGACCTTA TTACTGGGGT 24988
AAGGCAAGTC TGTTGACTGG TGTCCCTCAC CTCGCTTCCC TAATCTACAT TCAGGAGACC 25048
GAATCGGGGG TTAATAAGAC TTTTTTTGTT TTTTGTTTTT GTTTTTAACC TAGAAGAACC 25108
AAATCTGGAC GGCAAAACGT AGGCTTAGTT TGTGTGTTGT CTCTGAGTTT GTCGCTCATG 25168
CGTACAACAG GGTATGGACT ATCTGTATGG TGCCCCATTT TTGGCGGCCC GTAAGTAGGC 25228
TGGCTAGTCC AGGATACTGT GGAATAGCCA CCTCTTGACC AGTCATGCCT GTGTGCATGG 25288
ACTCAGGGCC ACGGCCTTGG CCTGGGCCAC CGTGACATTG GAAGAGCCTG TGTGAGAACT 25348
TACTCGAAGT TCACAGTCTA GGAGTGGAGG GGAGGAGACT GTAGAGTTTT GGGGGAGGGG 25408
TGGCAAGGGT GCCCAAGCGT CTCCCACCTT TGGTACCATC TCTAGTCATC CTTCCTCCCG 25468
GAAGTTGACA AGACACATCT TGAGTATGGC TGGCACTGGT TCCTCCATCA AGAACCAAGT 25528
TCACCTTCAG CTCCTGTGGC CCCGCCCCCA GGCTGGAGTC AGAAATGTTT CCCAAAGAGT 25588
GAGTCTTTTG CTTTTGGCAA AACGCTACTT AATCCAATGG GTTCTGTACA GTAGATTTTG 25648
CAGATGTAAT AAACTTTAAT ATAAAGGAGT CCTATGAACT CTACTGCTTC TGCTTCTTCT 25708
TCTCTGGACT GGTGGTATAG ATATAGCCAC CCTTTGCCCA AACCCTGGTA GCTCGGGGAA 25768
GCTTGGCTTA AGGCTGCACG CCTCCAATCC CCCAAAGGTA GGATCCTGGC TGGGTCCAGG 25828
GTTCCTCTGA TTTATTTGGT TTTGTTGTGT TGTGTTGTGT TTTTCTTTTG GCTAAACTTC 25888
TTTTGGAAGT TGGTAAGTTC AGCCAAGGTT TTACAGGCCC TGATGTCTGT TCTTCTAAAT 25948
GGTTTAAGTA ATTGGGACTC TAGCACATCT TGACCTAGGG TCACTAGAGC TAAGCTTGCT 26008
TTGCAGGGCA GACACCTGGG ACAGCCTTCC TCCCTCATGT TTGCTGGGAC ACTGCTGAGC 26068
ACCCCTTGCT TACTTAGCTC AGTGATGTTC CAGCTCCTGG CTAGGCTGCT CAGCCACTCA 26128
GCTAGACAAA AGATCTGTGC CCTGTGTTTC ATCCCAGAGC TTGTTGCCAG ATCACATGGC 26188
TGGATGTGAT GTGGGGTGGG GGTGGGGTCA TATCTGAGAC AGCCCTCAGC TGAGGGCTTG 26248
TGGGACAGTG TCAAGCCTCA GGCTGGCGCT CATTCATATA ATTGCAATAA ATGGTACGTG 26308
TCCATTTGGA CAGCAGACAC TTTGGTGTAC TTGTGCAGTC TCTTTTTGGT CTGGACCATG 26368
TCCAACTCTA TCTGGTTTTT GGAATGGGAG CCTAACTGGC CTGTGTTCTG GCTTGGTACC 26428
AAATAGCAAC AGTCAGTGGC ATCCTTGCCC AGGCCCCAGG GCAGGACTAT GCTCTTGCCA 26488
TATCCAGGAC TCCCGACTTT GCACCTGTTT TCCCTCTGTG TGTAGCATCA TGAACTCCAG 26548
CTAGGTTGTT CCTTTCCCTG GGGTCAGGAG GATTCTGCTG ACTCTGAATG TCAGGATTTG 26608
CTTTTGTTCT GTTTGCTTAT TGGGCAATTC TCAACCTTCA CTAGCAACAG TCTCATGTGT 26668

CAGGATTACA AGTATTGCTT GCACATTGAG GG 26700

(2) INFORMATION FOR SEQ ID NO: 2:

(i) SEQUENCE CHARACTERISTICS:
(A) LENGTH: 311 amino acids
(B) TYPE: amino acid
(D) TOPOLOGY: linear

(ii) MOLECULE TYPE: protein

(xi) SEQUENCE DESCRIPTION: SEQ ID NO: 2:

Met Arg Arg Ala Ala Leu Trp Leu Trp Leu Cys Ala Leu Ala Leu Arg
1               5                   10                  15

Leu Gln Pro Ala Leu Pro Gln Ile Val Ala Val Asn Val Pro Pro Glu
            20                  25                  30

Asp Gln Asp Gly Ser Gly Asp Asp Ser Asp Asn Phe Ser Gly Ser Gly
        35                  40                  45

Thr Gly Ala Leu Pro Asp Thr Leu Ser Arg Gln Thr Pro Ser Thr Trp
    50                  55                  60

Lys Asp Val Trp Leu Leu Thr Ala Thr Pro Thr Ala Pro Glu Pro Thr
65                  70                  75                  80

Ser Ser Asn Thr Glu Thr Ala Phe Thr Ser Val Leu Pro Ala Gly Glu
                85                  90                  95

Lys Pro Glu Glu Gly Glu Pro Val Leu His Val Glu Ala Glu Pro Gly
            100                 105                 110

Phe Thr Ala Arg Asp Lys Glu Lys Glu Val Thr Thr Arg Pro Arg Glu
        115                 120                 125

Thr Val Gln Leu Pro Ile Thr Gln Arg Ala Ser Thr Val Arg Val Thr
    130                 135                 140

Thr Ala Gln Ala Ala Val Thr Ser His Pro His Gly Gly Met Gln Pro
145                 150                 155                 160

Gly Leu His Glu Thr Ser Ala Pro Thr Ala Pro Gly Gln Pro Asp His
                165                 170                 175

Gln Pro Pro Arg Val Glu Gly Gly Gly Thr Ser Val Ile Lys Glu Val
            180                 185                 190

Val Glu Asp Gly Thr Ala Asn Gln Leu Pro Ala Gly Glu Gly Ser Gly
        195                 200                 205

Glu Gln Asp Phe Thr Phe Glu Thr Ser Gly Glu Asn Thr Ala Val Ala
    210                 215                 220

Ala Val Glu Pro Gly Leu Arg Asn Gln Pro Pro Val Asp Glu Gly Ala
225                 230                 235                 240

Thr Gly Ala Ser Gln Ser Leu Leu Asp Arg Lys Glu Val Leu Gly Gly
                245                 250                 255

Val Ile Ala Gly Gly Leu Val Gly Leu Ile Phe Ala Val Cys Leu Val
            260                 265                 270

Ala Phe Met Leu Tyr Arg Met Lys Lys Lys Asp Glu Gly Ser Tyr Ser
        275                 280                 285

Leu Glu Glu Pro Lys Gln Ala Asn Gly Gly Ala Tyr Gln Lys Pro Thr
    290                 295                 300

Lys Gln Glu Glu Phe Tyr Ala
305                 310

(2) INFORMATION FOR SEQ ID NO: 3:

     (i) SEQUENCE CHARACTERISTICS:
          (A) LENGTH: 2196 base pairs
          (B) TYPE: nucleic acid
          (C) STRANDEDNESS: both
          (D) TOPOLOGY: linear

    (ii) MOLECULE TYPE: DNA (genomic)

   (iii) HYPOTHETICAL: NO

   (iii) ANTI-SENSE: NO


    (xi) SEQUENCE DESCRIPTION: SEQ ID NO: 3:

TCTAGAACAC TTATTAAGAG CCAGGCACTG AAAAGTGCAG ACTCCCTCAT TTCATCCTGG      60

CCGTGCTTAC AAGTAGTTTC CATGCTCTGG TAACCCTGTG CAGAGGGCAG CGTGGGAGGC     120

GGGCCGCTTG GTGGACGGTC ATGGGGGCTC TGCATGGGTG GTTGCCCTTG CCTCAGAAGA     180

ACTCCCTAAG TAAGAGCAAG TTAGCCTCCC TAACCCCTGG TGGGTTGTTG CTTCTTTTCT     240

CCTCTTGTTT CTGCCAAGAG AGGGTGGACC AAGAAGACCC CAGCCTACAG AACATGTGAT     300

CCAAATAAAC TTCTTTTTAG TATAAATGTC CTAGCCTGTG ACGTTCTGGT AGACTAGCAC     360

AAGATGGACC AAGACAACTC TCATCGAGAC TCTGAGGAAC GAACTGGCAT CACATGGGAA     420

CAGGAAATGA AGCTTAGAGA GAGGTTCTGT GGCTTGTCCA ACATGGCTGT AGTTTAAATC     480

CAGCTTGCCA CCAAAGCACA CACATTTCAC TGCTGTGCTG GGCCGGGCCT CAGATCCCAG     540

GGGCTCCGGA GCTAGAAGGA CACGTGTATC AGCCATGGCT TCAGTTTATT GCTGTATACT     600

CTGTGCTTCT GGCTCTCATG GAAAAGACAG ACATTGGGGT TCTTATAATC TCTCCCTCTC     660

CCCTCCCCAC ACTCTATCCC CAAAGGAGGC ACCACTTCTG CAGGTAAATG TTATCTTCAA     720

AGCGCTCACA TCGCAACCTT TGCCCACACC ATCTCATTAA AGGAATTGGC AGTGACTTTA     780

AGGTGAAAGA ACTCGGTGGC TACGTGTTAT ATAAATTTGC ATCTGGGTCT CAGAGCTGGA 840
AGGAAGGCAC TCCCATACAT GCAGTCTGTA CATGCAGTCG GATGATGGAC CAACAACACA 900
TTGTGATTTA TGCCCCTGCT GGTGAGCCCA GGAATCCCTG TAGCACTCTC TCTCAGCTCT 960
AGGGCCCTGC TTGTGTATGG AAAACGCTTA GTGTTTTATA GGTATTTTGT CAGAATACTT 1020
TAAGGAACTT GACCAAAGTT ACAGGGAGGT TAGACAGATT GTCATGGTAT ACTCACCTCT 1080
GTCTCTGACC CTCCTAACTG GGACCTCTTT AGTCTCCCTT GAGGCAGGGA GTGCCACATG 1140
CATGTGTCCA GGCACATGTC TCCTGGTTTA CCTCCCAACG CACCTCAAGT CCCCAAGGTA 1200
GGTAGGCACT TGTATTCTGT AATTCAGAGA GGCAAATCAA ACTGTTACAA TGTTTGCCCA 1260
AAGCTCCCCA AGCAAAGTGG CCCTAAGAGT GAGCAAAGAG ACTGCGTGCC TTCACTGCCT 1320
GTGTGAATCC CTGCAGATAG TCTCTCATCT TGGTGCCCTT CCCACAGAGG CTGGGGCGGC 1380
AGGAGGGAGC CTGGACAGCT CAGACACTGG GTCATTGATG ACTGTTGTGT GGGATACCTG 1440
CCGGGGCGCA GGAGTGAGCC ATGCCACCCC AGGAAGTGGT TCAGGGTGAC TCTTCTTGGC 1500
ACACCTGGGA GGATGTAGCT GGTGCTGGCA CACCCACCGT CACGAGAGCT TCCTGTCCAA 1560
ACCTTCAACA AAGGCGGCTT CTTGAGACAG GCTAGACTGA AGTCACCAGC CTTGGGTGGG 1620
GTCCACTATG TAACCTCAGT GCTCAGGAAC CCTTTCCCAT ACTGTCTGGA ACTATACTGT 1680
ATGTAGCTGG GTTTCCACGC ATGTGTGCCT GCACCCAGTC CATCTCATCT TCTATCTCCC 1740
TCCCCTTTCC CGCTTCCCCC CTCCCCACTC TCCATCTCAT CTTCCATCCC CACCTCTTCT 1800
GGTCCCTGCC CTGCTAAACT CAGGGTAGCT GCATTCCGCT GGCCTTCCCC ATGTTCCAGG 1860
CTTCAGTCCC TTCTCTGCAC CTGTCCTTTG TGAAGTGACC AGAGGATTTC TGATCCTGTC 1920
TCTGTCGCTC TGAAGGGTCA GGAGTTCCTC CTGCCTGGAC AAAGCCATCC TGACGCACAT 1980
AAATAAAACA AACATCAAAC TCTATTCAAC CCCCTGGAAC CCGTGTGTGT TACTTACAGG 2040
GCAAAAGAAT GGAGCAGGGG ATGGGTTGTG GGGGGGGGGG GTGGCATCTG GGTTGTCTAC 2100
AGTTGTGCAT TAAGTTGTAA TTAAGATGTG CATTTCTCCA AATAAGGAAA AATTATTCTG 2160
GATTATTTGA GTGAAGCTGA AAGGTGATCA TCTAGA 2196

## Description

### FIELD OF THE INVENTION

This invention is in the field of cancer biology and therapy. Specifically, the invention is to a method for altering the differentiated state of a cell by altering syndecan expression. The method allows for the normalization of the growth rate and differentiation state of malignant cells, such method being based on the stimulation of syndecan expression in malignant cancer cells. Re-expression of syndecan in such malignant cells promotes their normal differentiated phenotype and prevents their tumor formation.

### BACKGROUND OF THE INVENTION

It is becoming more evident that cell surface proteoglycans play an important role in the regulation of cell behavior (Ruoslahti *et al., Cell 64*:867-869 (1991)). Through their covalently bound glycosaminoglycan side chains, such proteoglycans can bind various extracellular effector molecules (Jalkanen, *et al*., in *Receptors for Extracellular Matrix*, J. MacDonald & R. Mecham, Editors, Academic Press, San Diego, pp. 1-37 (1991)). One central challenge in proteoglycan biology is the need to understand what directive a cell receives by binding different effector molecules via the cell surface proteoglycans. It is further imperative to investigate what kind of intracellular response such binding activates, thereby leading to altered behavior of the cell. The way to approach these questions is to create biological models which are dependent on the expression of any given proteoglycans.

Syndecan is the best characterized cell surface proteoglycan (Saunders *et al., J. Cell Biol. 108*:1547-1556 (1989); Mali *et al., J. Biol. Chem*. *265*:6884-6889 (1990)). It was originally isolated from mouse mammary epithelial (NMuMG) cells as a hybrid proteoglycan containing both heparin sulfate and chondroitin sulfate glycosaminoglycan side chains (Rapraeger *et al., J. Biol. Chem. 260*:11046-11052 (1985)). Recent studies have revealed its expression, not only on epithelial cells but also on differentiating fibroblasts of developing tooth (Thesleff *et al., Dev. Biol. 129*:565-572 (1988); Vainio *et al., J. Cell Biol.* *108*:1945-1964 (1989)), on endothelial cells of sprouting capillaries (Elenius *et al., J. Cell Biol. 114*:585-596 (1991)) and on the surface of lymphocyte subpopulations (Sanderson *et al., Cell Regul. 1*:27-35 (1989)) intimating that its function can vary on the surfaces of different cells. Syndecan belongs to a family of proteoglycans with conserved plasma membrane and cytoplasmic domains but with dissimilar ectodomains (Mali *et al., J. Biol. Chem. 265*:6884-6889 (1990)). The conserved structure of syndecan suggests that it could participate in signal transduction through the plasma membrane.

Syndecan binds several extracellular effector molecules but this binding is selective. For example, syndecan binds interstitial collagens and fibronectin but does not bind to vitronectin or laminin (Koda *et al., J. Biol. Chem. 260*:8156-8162 (1985)); Saunders *et al., J. Cell Biol. 106*:423-430 (1988); Elenius *et al., J. Biol. Chem. 265*:17837-17843 (1990)). Moreover, syndecan isolated from tooth mesenchyme has revealed selective binding to tenascin not observed for syndecan from NMuMG cells (Salmivirta *et al., J. Biol. Chem. 266*:7733-7739 (1991)). This suggests variation in syndecan glycosylation that results in the selective binding properties for syndecan. Polymorphism of syndecan glycosylation has also been observed between simple and stratified epithelia (Sanderson *et al., Proc. Natl. Acad. Sci. USA 85*:9562-9566 (1988)); but whether these changes also reflect altered ligand recognition by syndecan remains unknown. Syndecan also binds growth factors, such as basic fibroblast growth factor (bFGF) (Kiefer *et al., Proc. Natl. Acad. Sci. USA 87*:6985-6989 (1990); Elenius *et al., J. Biol. Chem. 267*:6435-6441 (1992)). Very recently, Yayon and coworkers (Yayon *et al., Cell 64*:841-848 (1991)) and Rapraeger and co-workers (Rapraeger *et al., Science 252*:1705-1708 (1991)) have shown that heparin-like molecules are required for the binding of bFGF to its high affinity receptor, indicating that syndecan-like molecules can also modulate the growth factor response. The fact that cell surface proteoglycans can bind both growth factors and matrix components could theoretically imply a role in regulating, both temporally (timing of expression) and spatially (precise localization), growth promotion by immobilizing these effector molecules to the vicinity of cell-matrix interactions. This is supported by the intriguing pattern of syndecan expression in the development that follows morphogenetic rather than histological patterns (Thesleff *et al., Dev. Biol. 129*:565-572 (1988); Vainio *et al., J. Cell Biol. 108*:1945-1954 (1989) and Vainio *et al., Dev. Biol. 134*:382-391 (1989)), and moreover, that syndecan expression is localized to sites of active proliferation (Elenius *et al., J. Cell Biol . 114*:585-596 (1991) and Vainio *et al., Dev. Biol. 147*:322-333 (1991)).

In simple epithelium, syndecan is polarized to baso-lateral surfaces where it co-localizes with actin rich cytofilaments (Rapraeger *et al., J. Cell Biol. 103*:3683-2696 (1986)). Upon rounding, syndecan is shed from the cell surface by proteolytic cleavage of the core protein at the cell surface, a process which separates the matrix binding ectodomain from the membrane domain (Jalkanen *et al., J. Cell Biol. 105*:3087-3096 (1987)). By this way, syndecan has been proposed to be involved in the maintenance of epithelial morphology. Mouse mammary tumor cells (S115), when steroid-induced to modulate their morphology from an epithelial to a more fibroblastic or fusiform phenotype, lose syndecan expression (Leppä *et al., Cell Regul. 2*:1-11 (1991)), as to several other cell types *in vivo* when they transform (Inki *et al., Am. J. Pathol. 139*:1333-1340 (1991); Inki *et al., Lab. Invest. 66*:314-323 (1992)), suggesting a general loss of syndecan expression during malignant transformation.

### SUMMARY OF THE INVENTION

It is therefore an object of the subject invention to provide a method of altering the differentiated state of a host cell by aftering syndecan expression in such cell.

It is a further object of the invention to provide a method to induce and regulate syndecan expression, especially in cells which exhibit a malignant phenotype, regardless of the origin of transformation.

It is a further object of the invention to provide a treatment for the reduction of tumor growth in a patient in need of such treatment, by administration of a composition to such patient, such composition comprising efficacious amounts of one or more agents that stimulate syndecan synthesis in the tumor cells of such patient.

It is a further object of the invention to provide the DNA sequence and localization of promoter, suppressor and enhancer elements for the syndecan gene.

It is a further object of the invention to provide a method for the enhancement of syndecan expression in a host cell, by enhancing syndecan gene transcription.

It is a further object of the invention to provide a method for the enhancement of syndecan expression in malignant cells, by preventing suppression of syndecan gene transcription.

It is a further object of the invention to provide a biochemical method for the inactivation of suppressors of syndecan gene expression in malignant cells.

Further features, objects and advantages of the present invention will become more fully apparent from a detailed consideration of the following description of the subject invention when taken together with the accompanying drawings.

### BRIEF DESCRIPTION OF THE DRAWINGS

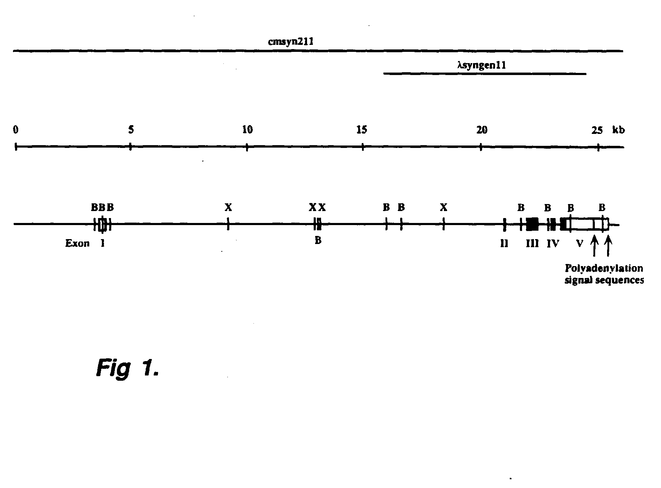

Figure 1. Figure 1 is a diagram of the assembly of mouse syndecan gene and its promoter region.

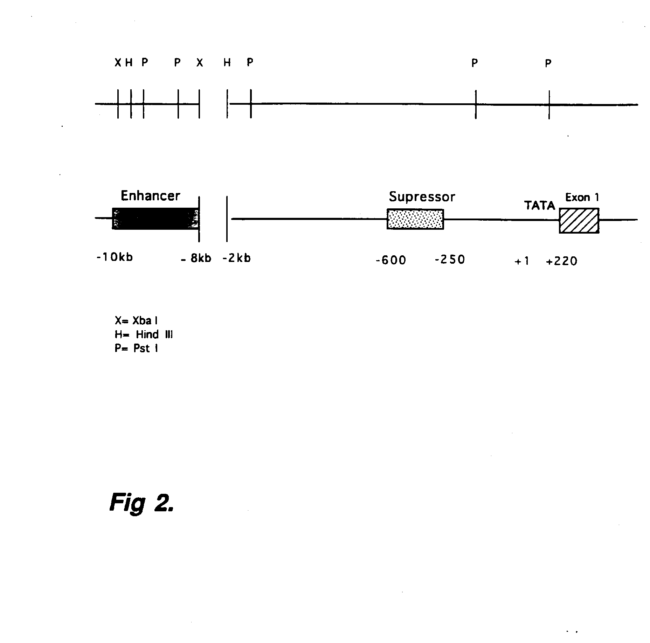

Figure 2. Figure 2 is the complete sequence of the mouse syndecan gene [SEQ ID Nos. :1: (DNA) and :2: (protein)] and its proximal promoter. Regulatory sites for the expression of syndecan may also exist on the first intron following the first exon (see Figure 1).

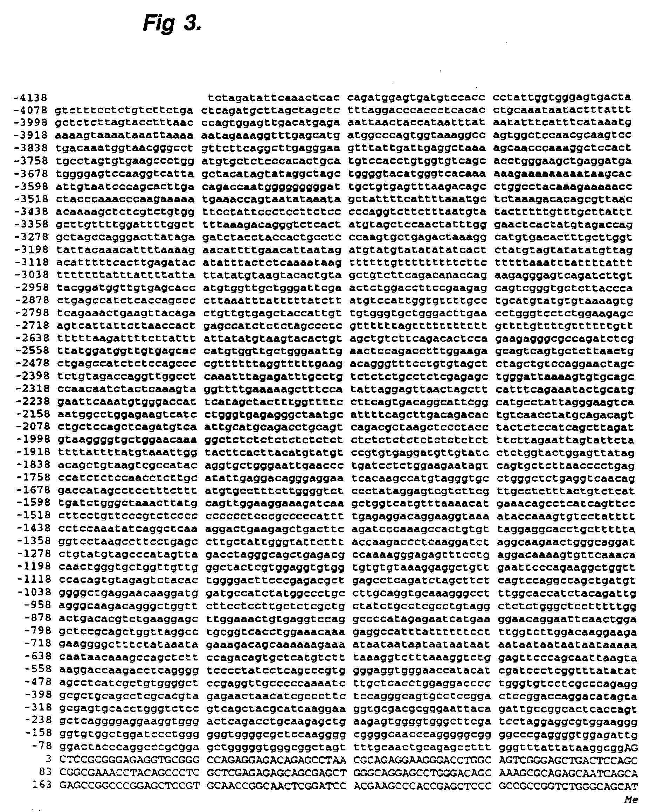

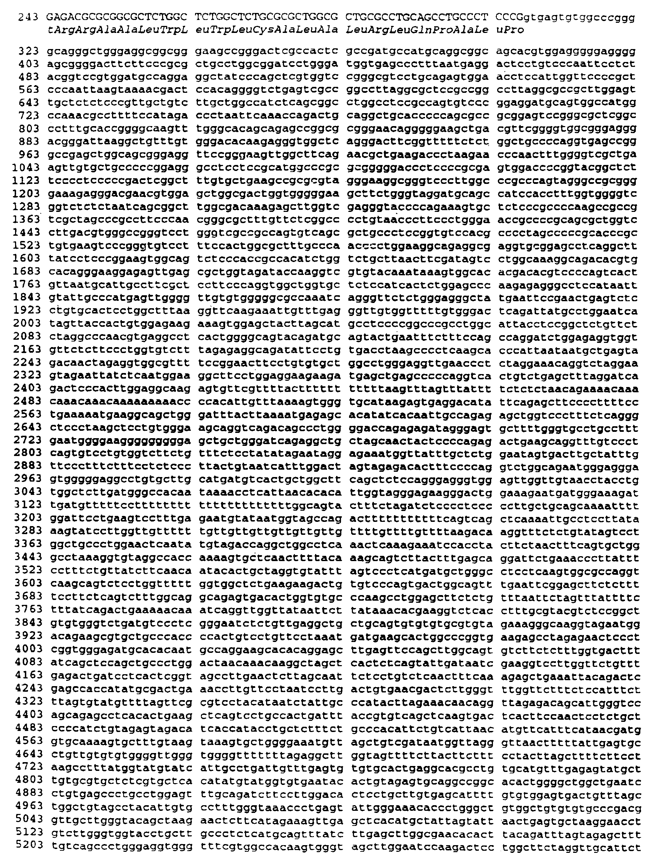

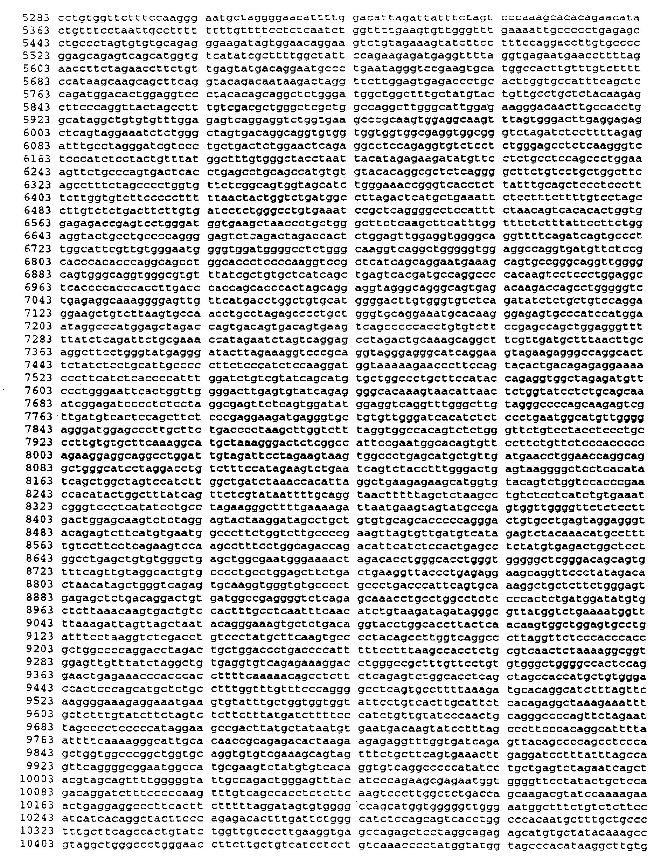

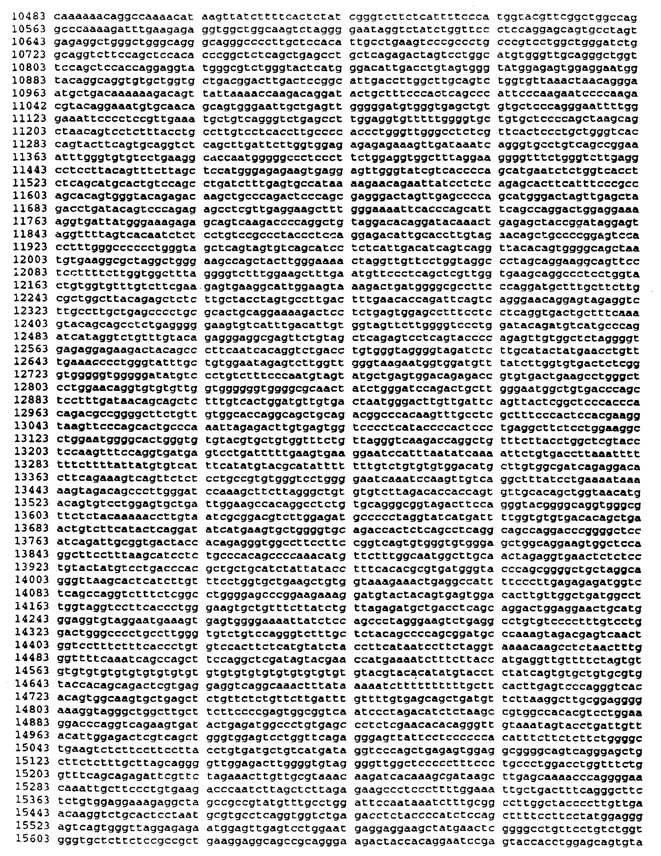

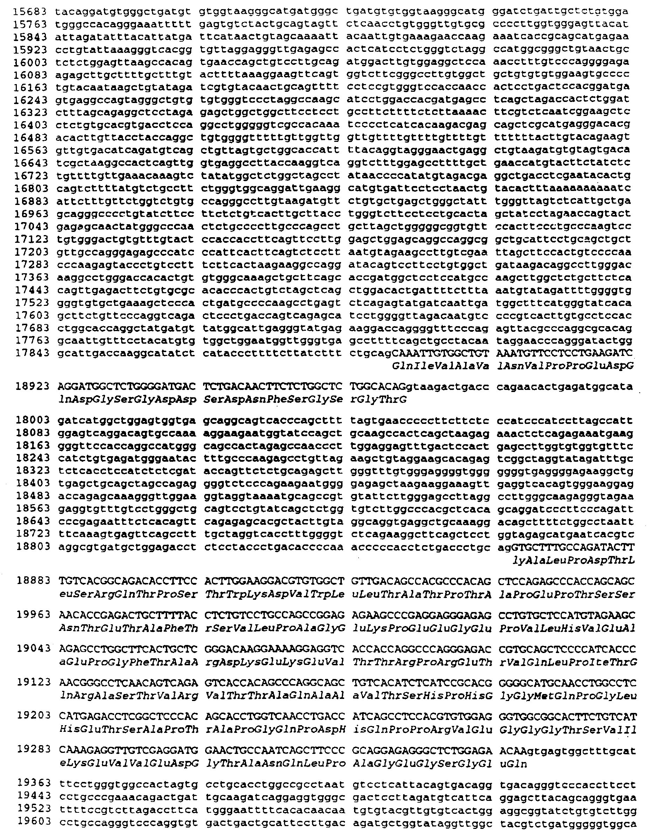

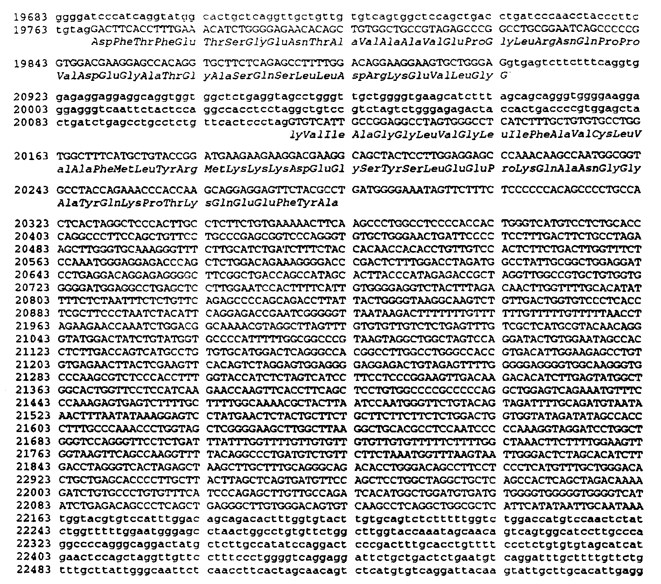

Figure 3. Figure 3 is a diagram of the assembly of mouse syndecan promoter region and the localization of the enhancer and suppressor elements together with restrictions sites for three different enzymes.

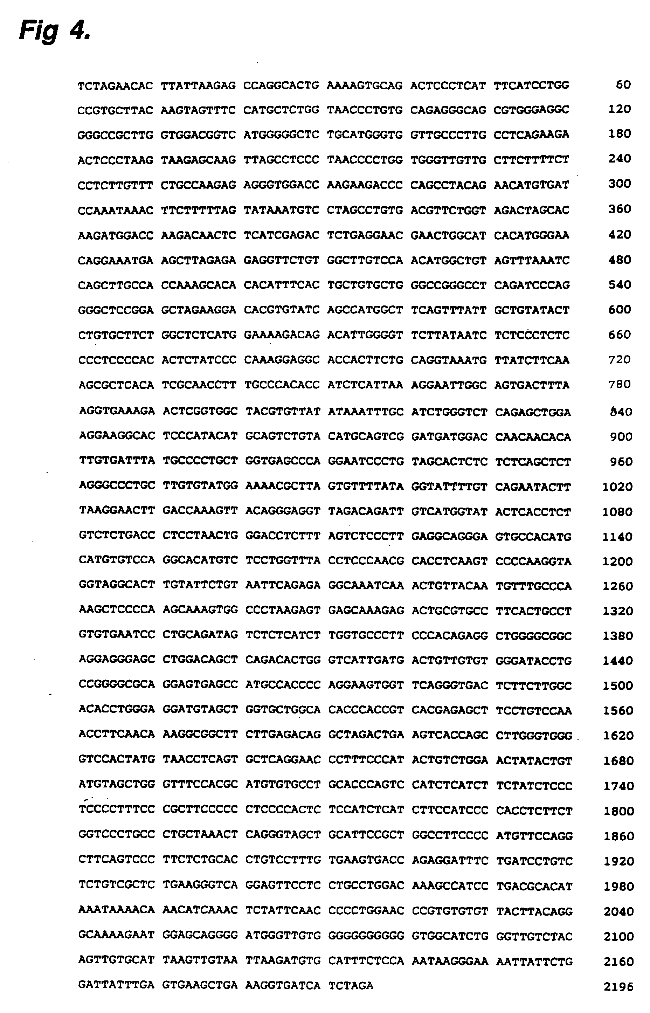

Figure 4. Figure 4 is the complete sequence of the mouse syndecan enhancer element [SEQ ID No. :3: (DNA)].located 8-10 kbs upstream from the transcription initiation site as indicated in figure3.

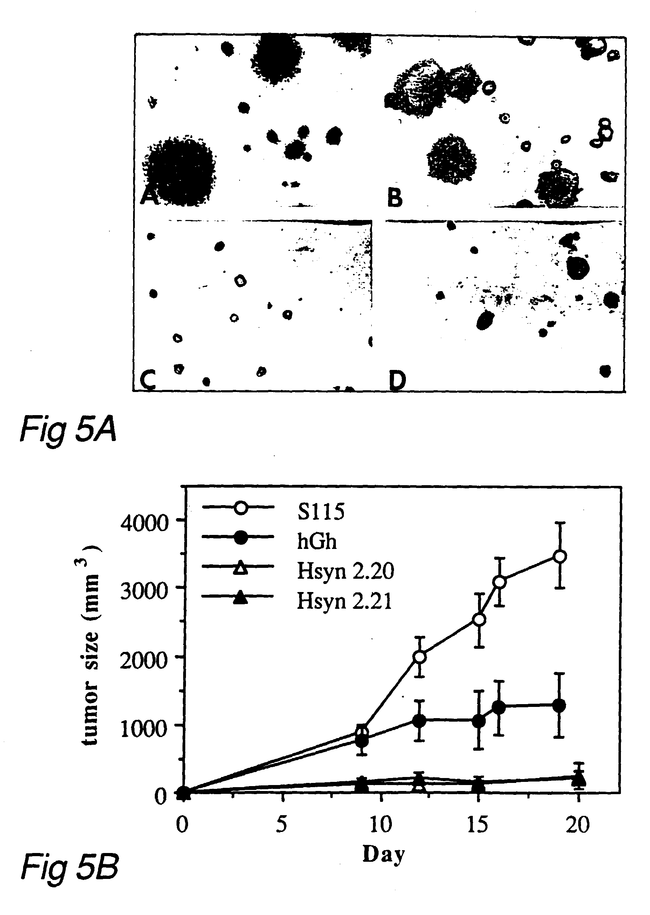

Figure 5. Figure 5A (panels A-D) is a photographic presentation of reduced growth ability of syndecan-transfected cells in soft agar. Panel A (wild-type S115 cells) and B (control transfected cells) are pictures of the colonies that are formed in soft agar in the presence of testosterone, a feature typical for hormone-transformed cells. This growth ability was not observed with two independent syndecan-transfected cell clones (panels C and D), demonstrating how syndecan re-expression can overcome the effect of hormone-induced transformation. Figure 5B is a graphical presentation of how syndecan-transfected cells lose their ability to form tumors in nude mice. Wild-type or control transfected cells produce tumors in testosterone-administered nude mice while syndecan transfected cells revealed a very low tendency to produce tumors.

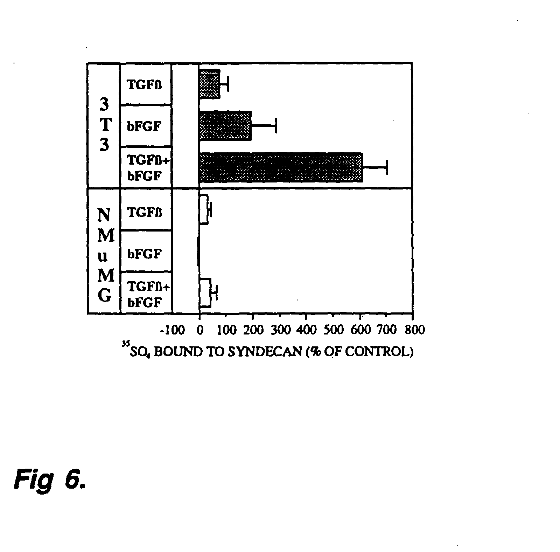

Figure 6 is a graphical representation of enhanced syndecan expression in 3T3 cells by simultaneously administered basic fibroblast growth factor (bFGF) and transforming growth factor beta (TGF-β). This is an example of how syndecan expression can be enhanced as a result of growth factor action in normal cells during the differentiation process.

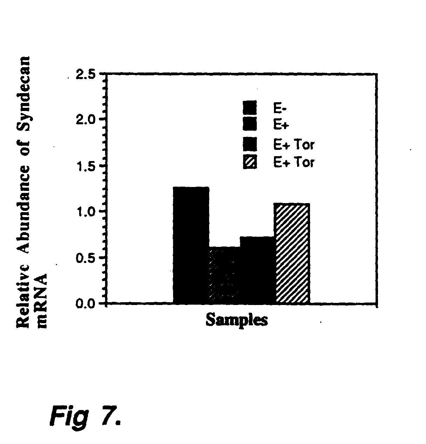

Figure 7 is a graphical representation of enhanced syndecan expression by MCF-7 cells exposed to the anti-estrogen toremifene. When exposed to estrogen, syndecan expression In MCF-7 cells was reduced and the cells transformed. Subsequent treatment with the anti-estrogen (toremifene) restored syndecan expression to levels close to that found in cells not exposed to estrogen and aided the cells in maintaining their normal growth behavior.

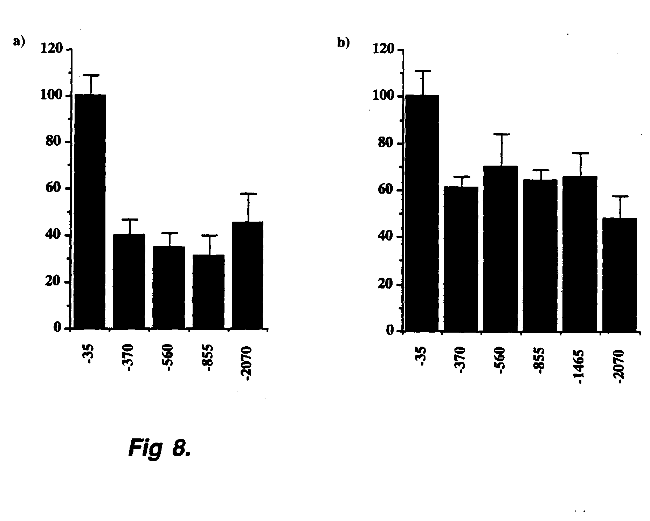

Figure 8 is a graphical presentation of how the suppressor element (see figure 3) is acitve in S115 cells treated with testosterone. Indicated stretches of promoter sequences were transfected in hormone-treated S115 cells and analysed for their transcription activity as described in Example VI. A dramatic drop was observed wiht suppressor construct as indicated in Figure 3, and it was more obvious in transformed S115 (a) cells than in 3T3 cells (b).

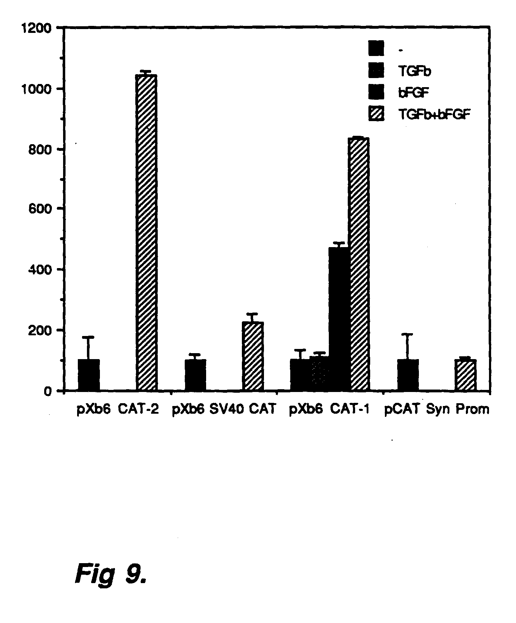

Figure 9 is a graphical presentation of how the enhancer element is active in growth hormone-treated 3T3 cells. Various stretches of promoter were transfected in 3T3 cells and analysed for their transcription activities. Fragment pXb6, which is the same as illustrated in Figure 3 as an enhancer, revealed more than ten fold stimulation in 3T3 cells exposed to growth factors bFGF and TGFβ if compared to non-treated cells.

### DEFINITIONS

In order to provide a clearer and more consistent understanding of the specification and claims, including the scope to be given such terms, the following definitions are provided.

"Enhancement" or "Stimulation" of Syndecan expression. By "enhancement" or "stimulation" of syndecan expression" is meant an effect of increasing the synthesis of syndecan, either by the induction or de-suppression (de-repression) of syndecan gene transcription and/or translation.

Cell growth. By "cell growth" is meant cell replication, both controlled and uncontrolled.

Malignant. By "malignant" is meant uncontrolled cell growth.

More Differentiated Phenotype. In stating that a cell has a "more differentiated phenotype" is meant that the cell possesses a phenotype usually possessed by a certain cell type more differentiated than the cell, which the cell was deficient in prior to enhancement of syndecan expression according to the invention. This phenotype may be defined by one or more phenotypic characteristics. For example, an epithelial cell is a more differentiated phenotype of a mesenchymal-like shape; therefore, the ability of the method of the invention to maintain cells in an epithelial cell morphology rather than a mesenchymal-like shape is a more differentiated phenotype within the meaning of the definition. Continuous syndecan expression is necessary for the maintenance of terminal differentiation of epithelial cells.

Syndecan expression Is also linked to the normal differentiation of mesenchymal cells. However, only a transient increase in syndecan expression is needed for normal differentiation in mesenchymal cells. Therefore, contrary to epithelial cells, expression of syndecan is not needed for maintenance of terminal differentiation in mesenchymal cell. To induce differentiation of a suitable mesenchymal precursor cell population (such as a "condensing mesenchymal" cell population) to a fully differentiated mesenchymal cell, there is needed only a transient expression of syndecan expression. Therefore, a terminally differentiated mesenchymal cell is a "more differentiated phenotype" than a condensing mesenchymal cell.

Other useful phenotypes that are present in syndecan-deficient cells and not in cells expressing sufficient syndecan include fusiform shapes with long filopodial extensions with extensive under-and overlapping of these processes (so that the cells appear to have a detect in cell adhesion).

In another example, syndecan-deficient NMuMG cells continue to secrete milk fat globule antigen (and thus appear mammary-like) and continue to express cytokeratins (thus appear epithelial-like). However, their actin-containing cytoskeleton is disorganized and their expression of beta₁ integrins and E-cadherins at the cell surface is markedly reduced. Upon expression of sufficient syndecan, these phenotypes are corrected so that there is no reduction in cell surface integrins or E-cadherin and the cell has an epithelial morphology. Therefore, the amount of cell surface integrins or E-cadherin are useful markers of syndecan expression and may be used to monitor what amount of a drug is needed for efficacious results according to the method of the invention.

Efficacious Amount. An "efficacious amount" of an agent is an amount of such agent that is sufficient to bring about a desired result, especially upon administration to an animal or human.

Administration. The term "administration" is meant to include introduction of agents that induce syndecan expression into an animal or human by any appropriate means known to the medical art, including, but not limited to, injection, oral, enteral and parenteral (e.g., intravenous) administration.

Pharmaceutically Acceptable Salt. The term "pharmaceutically acceptable salt" is Intended to include salts of the syndecan-inducing agents of the invention. Such salts can be formed from pharmaceutically acceptable acids or bases, such as, for example, acids such as sulfuric, hydrochloric, nitric, phosphoric, etc., or bases such as alkali or alkaline earth metal hydroxides, ammonium hydroxides, alkyl ammonium hydroxides, etc.

Pharmaceutically Acceptable Vehicle. The term "pharmaceutically acceptable vehicle" is intended to include solvents, carriers, diluents, and the like, which are utilized as additives to preparations of the syndecan-inducing agents of the invention so as to provide a carrier or adjuvant for the administration of such compounds.

Treatment. The term "treatment" or "treating" is intended to include the administration of compositions comprising efficacious amounts of syndecaninducing agents to a subject for purposes which may include prophylaxis, amelioration, prevention or cure of a medical disorder, including tumor growth, hair growth.

### DETAILED DESCRIPTION OF THE PREFERRED EMBODIMENTS

Briefly, in its broader aspects the present invention comprehends a method for maintaining a differentiated phenotype in a normal (non-malignant) cell that otherwise would suppress syndecan expression, by maintaining syndecan expression in such cell. The invention also comprehends a method for inducing a more differentiated phenotype in a malignant cell that lacks (or is deficient in) syndecan expression, by stimulating syndecan expression in such a cell. As used herein, if a cell is said to "lack" syndecan expression, it is meant to include cells that are either completely deficient in syndecan protein or produce insufficient syndecan levels to maintain or attain a desired differentiated phenotype.

The subject method may be accomplished in a variety of manners including biochemical, chemical or even molecular biological type methods. While the method is applicable to a variety of cancer (both malignant and non-malignant) and normal cells, it is particularly adaptable for treating malignant cells which have become transformed. This includes cells transformed due to hormonal influences of the body or environmental influences, such as chemicals or radiation exposure. Especially, the tumor type is a tumor characterized by loss of syndecan, for example, a glioma, myeloma, carcinoma, sarcoma, lymphoma, or adenoma.

Generally, any cell genetically capable of expressing syndecan can be a cell stimulated to express syndecan by the method of the invention. Syndecan is naturally expressed in a wide variety of epithelial cells in mature and embryonic tissues and by various embryonic mesenchymal tissues undergoing inductive interactions with epithelia. In addition, syndecan is naturally expressed on Leydig cells and on developing B-lymphocytes and a subpopulation of plasma cells.

Enhanced syndecan expression may be achieved by administration of compositions containing a biochemically, and/or chemically and/or molecular-biologically active component to an individual. Compositions may be administered orally, intravenously, subcutaneously or locally, or by any other method which will allow cells, normal or malignant, to be exposed to syndecan expression enhancing component.

By a "biochemically" or "chemically" active component is meant a component that alters the endogenous syndecan biochemistry or chemistry of the target cell without altering syndecan gene expression *per se*. For example, such alterations may include altering the half-life of syndecan protein or mRNA, so as to increase levels of syndecan protein in the cell, for example, by altering the external domain of the cell's endogenous syndecan, or the cell surface membrane properties in general, so as to retain higher levels of syndecan on the cell surface; and, altering the syndecan protein active site(s), so as to enhance the efficiency of the syndecan response.

By a "molecular-biologically' active component is meant a component that alters endogenous syndecan gene expression in a manner that allows for an increase in cellular syndecan, such as, for example, by stimulating transcription, preventing (or reducing) suppression of transcription, derepression transcription, and generally increasing levels of mRNA and/or translation efficiency.

It is known that cellular transformation involves activation of some cell growth stimulating genes (like oncogenes) and inactivation of some other genes, which work to suppress cell growth. It has recently been shown that loss of syndecan expression is observed upon transformation of cells, and that this suppression is due to syndecan gene inactivation (Leppä *et al., Cell Regul. 2*:1-11 (1991); Inki *et al., Am. J. Pathol.* 139:1333-1340 (1991); Inki *et al., Lab. Invest. 66*:314-323 (1992)). This was demonstrated in several biological models of various known carcinogenic systems (oncogenes, chemical carcinogens, UV-light, hormone-exposure, etc.). Therefore, syndecan gene suppression is implicated in the development of cellular transformation.

Further, according to the invention, all the manipulations of such cells which can induce syndecan expression in malignant cells, also induce these cells to obtain a more differentiated phenotype, and thus, subsequently reduce their potential tumorigenic behavior and metastasis.

In a preferred embodiment, the cell in which syndecan expression is stimulated is a cell that Is steroid-responsive. Examples of such steroid-responsive cells include breast cells, endometrium cells and prostate cells, especially in the malignant state. In a highly preferred embodiment, the cell is responsive to estrogen and/or androgen.

Examples of other cell types that will respond to the treatment of the invention include malignant and non-malignant mesenchymal cells.

The regulatory elements of a given gene are commonly located upstream from (5' to) the transcription initiation site. Syndecan, however, has a very peculiar gene structure, in which the first and second exons are separated by a very large intron (Figure 1). This could mean that, in addition to the base sequences upstream from the transcription site, syndecan expression may also be susceptible to regulation by base sequences located between first and second exons (Figure 2), that is, in the first intron.

It has now surprisingly been found that the syndecan gene has a strong enhancer element located 8-10 kb upstream from the transcription initiation site. The sequence of this enhancer element has been identified and is given in Figure 4 [SEQ ID. No. 3].

Manipulation of the upstream region of the syndecan gene can block its inactivation during malignant transformation. For example, replacement of the region in front of first exon of the syndecan gene with the glucocorticoid-inducible elements of mouse mammary tumor virus (MMTV) not only blocks syndecan suppression during malignant transformation, but also inhibits the ability or potential of cells to transform and become tumorigenic (Figure 3). These findings suggest a very important role for syndecan in the maintenance of normal epithelial morphology (Leppä *et al., Proc. Natl. Acad. Sci. USA 89*:932-936 (1992)).

Cells destined to differentiate during organ formation or tissue regeneration also exhibit enhanced syndecan expression (Vainio *et al., Dev. Biol. 147*:322-333 (1991); Elenius *et al., J. Cell Biol. 114*:585-595 (1991)). The component(s) responsible for the regulation of syndecan expression (either directly or indirectly) have not yet been identified. Growth factors are candidates for this role since they are known to be involved in the regulation of early development and cellular differentiation (Heath *et al., Curr. Opin. Cell Biol. 3*:935-938 (1991)). Involvement of growth factors is also supported indirectly by the fact that the expression of two embryonally important growth factors (TGF-β and FGF) has been shown to coincide with syndecan within developing tooth (Vaahtokari *et al., Development 113*:985-994 (1991); Wilkinson *et al., Development 105*:131-136 (1989)).

Based on these findings the possible effect of growth factors on the expression of syndecan has been tested. It was shown that both bFGF and TGF-β alone, but especially if administered in combination, enhance syndecan expression by 3T3 cells (Figure 4). This stimulation is close to the levels observed in syndecan-expressing epithelial cells (Elenius *et al., J. Biol. Chem. 267*:6435-6441 (1992)) prior to their malignization (Leppä *et al., Proc. Natl. Acad. Sci. USA 89*:932-936 (1992)). These findings suggest that growth factors, and their derived fragments and domains may prove to be valuable for the development of an active tool for the regulation of syndecan expression.

Preferably, for treatment of humans and animals, a drug is administered that results in the enhancement of syndecan expression to levels sufficient to facilitate cellular differentiation in the degenerative stages of tissues. Such drugs are herein termed "syndecan-inducing agents." Syndecan-inducing agents include the growth factors and their derivatives that retain growth-factor activty. Examples of such growth factors include bFGF, and TGF-β, whether administered separately or together.

Even more preferred us a syndecan-inducing agent that has good tissue and cell penetration so that it could directly interfere with suppressor(s) of syndecan expression within cell nuclei. Such a syndecan-inducing agent is the known antitumor drug toremifene. When toremifene, known to have good plasma membrane penetration, is administered to the hormone-transformed epithelial cells with reduced syndecan expression, the cells reverse their lowered syndecan expression, and induce syndecan levels to those close to the level observed with normal, non-transformed cells (Figure 5). This demonstrates that syndecan-inducing agents useful in the methods of the invention are known and available and that such agents can specifically prevent cellular malignization by blocking suppression of syndecan expression. Another useful drug in this regard is tamoxifen.

Such syndecan-inducing agents may be administered using currently available preparations, or in any pharmaceutically acceptable vehicle. The route of administration may be any route that delivers efficacious levels of the drug to the desired active site, for example, by injection.

For parenteral administration, preparations containing one or more syndecan-inducing agents may be provided to the patient in need of such treatment in combination with pharmaceutically acceptable sterile aqueous or non-aqueous solvents, suspensions and emulsions. Examples of non-aqueous solvents are propylene glycol, polyethylene glycol, vegetable oil, fish oil, and injectable organic esters. Aqueous carriers include water, water-alcohol solutions, emulsions or suspensions, Including saline and buffered medical parenteral vehicles including sodium chloride solution, Ringer's dextrose solution, dextrose plus sodium chloride solution, Ringer's solution containing lactose, or fixed oils. Intravenous vehicles include fluid and nutrient replenishers, electrolyte replenishers, such as those based upon Ringer's dextrose and the like.

The syndecan-inducing agent of the invention can be employed in dosage forms such as tablets, capsules, powder packets, or liquid solutions for oral administration If the biological activity of the syndecan-inducing agent is not destroyed by the digestive process and if the characteristics of the compound allow it to be absorbed across the intestinal tissue.

The syndecan-inducing agents may also be administered by means of pumps, or in sustained-release form. The syndecan-inducing agents used In the method of invention may also be delivered to specific organs in high concentration by means of suitably Inserted catheters, or by providing such molecules as a part of a chimeric molecule (or complex) which is designed to target specific organs.

Administration in a sustained-release form is more convenient for the patient when repeated injections for prolonged periods of time are indicated.

The composition containing the syndecan-inducing agent can be manufactured in a manner which is in itself know, for example, by means of conventional mixing, granulating, dragee-making, dissolving, lyophilizing or similar processes. The compositions of the present invention that provide the syndecan-inducing agent, in and of themselves, find utility in their ability to slow or prevent tumor growth or tumor reappearance. The syndecan-inducing compositions of the invention utilize the body's own mechanisms for promoting differentiation of specific cell types to its maximum potential.

In intravenous dosage form, the compositions of the present invention have a sufficiently rapid onset of action to be useful in the acute management of tumor growth.

Additionally, a low potency version is useful in the management of disorders wherein a tumor has been effectively treated and the patient appears to be in remission, but it is desired to maintain sufficient levels of syndecan-inducing agents in the patient so as to assist the body in preventing a recurrence of the tumor.

Typical doses of toremifene or tamoxifen, and other such syndecan-inducing agents useful in the methods of the invention for treatment of humans or other animals are 20-600 mg daily, and preferably 20-60 mg daily.

The examples below are for illustrative purposes only and are not deemed to limit the scope of the invention.

### EXAMPLE I

### Reversal of hormone-induced transformation by exogenous syndecan expression.

As previously described (Leppä *et al., Cell Regulation 2*:1-11 (1991)), S115 mouse mammary tumor cells were routinely cultured in DMEM. For experimental studies involving hormone treatment, inactivated fetal calf serum (i-FCS) was replaced with 4% dextran charcoal-treated fetal calf serum (DCC-FCS), which eliminates endogenous steroids from serum), and used either with or without testosterone (10 nM) and with or without dexamethasone (10 nM or 1 µM). Cells were plated at a density of 10,000 cells/cm² and the medium was replenished every 3 days.

Plasmid pUC19-hsynpr7 containing human syndecan cDNA (Mali *et al., J. Biol. Chem. 265*:6884-6889 (1990)) was digested with *Nae*I restriction endonuclease, and the derived 336 bp long-fragment was separated in and eluted from low melting agarose gel. Plasmid pUC19-hsyn4 (Mali *et al., J. Biol. Chem. 265*:6884-6889 (1990)) was digested with *Nae*I and *Hind*II (polylinker site), and the plasmid-containing fragment starting from base 487 was isolated. The *Nae*I fragment from hsynpr7 was ligated to the pUC-hsyn *Nae*I/*Hind*II digested vector. The orientation of insert was verified by restriction enzyme analysis and sequencing. The derived plasmid, containing the full coding region of human syndecan core protein, was named pUC19-hsynfull. This plasmid was further digested with *Bam*HI and *Sph*I (polylinker site). A fragment containing syndecan coding region bases 150-1461 was isolated and blunt-ended, using Klenow and T4 DNA polymerase. Finally, this fragment was ligated to *Sal*I-linearized and blunt-ended pMAMneo vector (Clontech; Palo Alto, CA), resulting in a chimeric gene containing RSV-MMTV-LTR promoter connected to human syndecan coding region and SV-40 polyadenylation signal. The orientation was confirmed by restriction enzyme digestions. The plasmid was named pMAMneo-hsyn.

For control transfections, a 642 bp long-*Hind*III/*Pvu*II fragment of human growth hormone gene (consisting of exons 4 and 5; Bornstein *et al., J. Biol. Chem. 263*:1603-1606 (1988)) was blunt-ended and cloned into the same pMAMneo vector, as described above. This control construct was named pMAMneo-hGH.

All plasmids were isolated using the CsCl density gradient method. Before transfections, plasmids were linearized with *Mlu*I, chloroform/phenol extracted and ethanol precipitated.

Transfections were performed using Upofectin™ (BRL), according to manufacturer's instructions. After selection for two weeks (G418; 750 µg/ml, Sigma), surviving clones were isolated from growth plates using cloning cylinders. The expression of human syndecan or growth hormone (consisting of exons 4 and 5) mRNAs was then confirmed by RNA isolation and Northern blot analysis. Clones expressing high levels of transfected genes were selected for further studies and characterizations. These stock cells were routinely cultured in the presence of G418 (300 µg/ml).

For the measurement of exogenous syndecan expression total RNA was isolated from wild-type S115 cells and cells transtected with human syndecan or growth hormone genes. RNA was extracted using 4M guanidine isothiocyanate and CsCl pelleting, as earlier described by Chirgwin *et al., Biochemistry 18*:5294-5299 (1979)). RNA from normal mouse mammary NMuMG and normal human mammary HBL-100 cells was used for comparison. RNA aliquots of 15 µg were separated in 1% formaldehyde agarose-gel electrophoresis and transferred to GeneScreen Plus™ hybridization membrane (New England Nuclear). Blots were hybridized with multiprime (Amersham) labeled inserts of either mouse (PM-4) (Saunders *et al., J. Cell Biol. 108*:1547-1556 (1989)) or human syndecan (pUC19-hsyn4 *Bam*HI 1.1 kb fragment) (Mali *et al., J. Biol. Chem. 265*:6884-6889 (1990)), or with human growth hormone exons 4 and 5 (hGH) (Leppä *et al., Proc. Natl. Acad. Sci. USA 89*:932-936 (1992)) cDNAs, using the high stringency conditions suggested by the manufacturer of the membrane (New England Nuclear). All techniques based on modem molecular biology are fully explained in the literature such as in the laboratory manual entitled *Current Protocols in Molecular Biology*.

Anchorage independent cell growth was measured in a soft agar colony assay. The six well-plates were first covered with an agar layer consisting of 2 ml DMEM, 0.5% agar and 4% DCC-FCS. The middle layer contained 10⁴ cells in 0.5 ml DMEM supplemented with 0.33% agar and 4% DCC-FCS, with or without 10 nM testosterone. The uppermost layer, consisting of medium (2 ml), was added to prevent drying of the agarose gels. The plates were incubated at 37°C in 5% CO₂ for 12 days after which cultures were evaluated and photographed.

Tumorigenicity of S115 wild type cells, one hGH transfected control clone and two clones expressing human syndecan-1 was measured in nude mice. For this cells were cultured in DMEM containing 5% FCS and 10 nM testosterone. After four days in culture, cells were harvested with trypsin, washed, and 10⁷ cells suspended in 0.2 ml of DMEM were injected subcutaneously into back of athymic male nude mice (balb-C). Silastic testosterone capsule, which is known to increase the growth rate of S115 cells (King *et al., J. Steroid. Biochem. 7*:869-873 (1976)) was simultaneously implanted. Nude mice were examined regularly for tumor development and the size of the palpable tumors measured at intervals.

### EXAMPLE II

### Growth factors enhance syndecan expression.

NMuMG mouse mammary epithelial cells and 3T3 (NIH) mouse fibroblasts were routinely cultured in bicarbonate-buffered Dulbecco's modified Eagle's medium (DMEM; GIBCO) containing 10% FCS (GIBCO) and antibiotics, as previously described (Elenius *et al., J. Biol. Chem. 265*:17837-17843 (1990)). For experiments, cells were plated at equal density on culture dishes (Nunc) and grown to 60 - 70% confluency. Twenty-four hours before supplementing growth factor(s) to the medium, fresh medium containing 2% CMS-FCS (Vogel *et al, Proc. Natl. Acad. Sci. USA 75*:2810-2814 (1978)) was replaced. Equally treated cultures without growth factor addition served as negative controls. Porcine TGFβ1 (R&D), recombinant human bFGF (Boehringer) and murine EGF (Sigma) were used in final concentrations of 2.5 ng/ml (100 pM), 10 ng/ml (570 pM) and 1.2 ng/ml (200 pM) respectively, in all experiments. For quantitation and isolation of cell surface syndecan, media were discarded at time points indicated in the text and the cell layers were washed twice with ice cold phosphate buffered saline (PBS). Cells were scraped with a rubber policeman into ice cold PBS supplemented with 0.5 mM EDTA and centrifuged. After subsequent washes by resuspension and centrifugation the cell numbers were measured by counting the nuclei with a Coulter Counter (Coulter Electronics).

For quantitation of syndecan intercalated into the cell membrane, syndecan ectodomain was released by incubating washed cells in 20 µg/ml bovine pancreatic trypsin (Type III; Sigma) in PBS for 10 min on ice bath. After incubation the cells were centrifuged, leaving the ectodomain in the supernatant (Rapraeger *et al, J. Biol. Chem. 260*:11046-11052 (1985)). Sample volumes equal to 400,000 or 200,000 cells for 3T3 or NMuMG cells, respectively, were loaded on cationic nylon membrane (Zeta-Probe; BioRad) in a minifold-slot apparatus (Schleicher and Schuell), as previously described (Jalkanen *et al., J. Cell Biol. 105*:3087-3096 (1987)). Nonspecific binding was blocked by incubating the membrane for one hour at room temperature in PBS containing 10% FCS. Syndecan attached to the membrane was detected with a monoclonal antibody against mouse syndecan core protein (mAB 281-2) (Jalkanen *et al., J. Cell Biol. 101*:976-984 (1985)) that was radioiodinated by chloramine-T oxidation method (Stähli *et al., Meth. Enzymol. 92*:242-253 (1983)). The membrane was incubated overnight at 4°C with ¹²⁵I-labeled 281-2 in PBS + 10% FCS (10,000 CPM/ml). After five washes with PBS the bound antibody was visualized by autoradiography.

The accumulation of syndecan ectodomain into the medium was estimated by taking samples corresponding to 1/50 (3T3 cells) or 1/100 (NMuMG cells) of the total volume of the remaining medium at selected time points. The samples were analyzed by loading them to nylon membrane as described above. The autoradiography signal was quantitated with a GelScan XL ultroscan densitometer (LKB) using GelScan XL 2400 software (LKB).

For syndecan purification, cells were radiolabeled for 24 hours in low sulfate DMEM (MgCl₂ substituted for MgSO₄; 2% CMS-FCS) with 100 µCi/ml ³⁵SO₄ (New England Nuclear) in the presence or absence of growth factor(s). Cell surface trypsin-releasable material was collected, as described above, and after dialysis against Tris-buffered saline (TBS), the sample was loaded onto a 281-2-Sepharose CL-4B immunoaffinity column (Jalkanen *et al., J. Cell Biol. 105*:3087-3096 (1987)). Bound material was eluted with 50 mM triethylamine (TEA) (pH 11.5) and the amount of radioactive PG in each fraction was analyzed using cetylpyridiumchloride-impregnated Whatman 3MM filter discs (Rapraeger *et al., J. Biol. Chem. 260*:11046-11052 (1985)). For interaction experiments, fractions containing most of the labeled PG were pooled and dialyzed against PBS.

To obtain unlabeled syndecan ectodomain for interaction assays (see below) the same procedure was used except that no radioactive sulfate was added to the culture medium and the syndecan containing fractions eluted from the immunoaffinity column were detected by immuno-dot assay using mAB 281-2. The estimation of the molar concentration of syndecan was based on the use of previously determined syndecan concentration by total amino acid analysis (Jalkanen *et al., J. Cell Biol. 106*:953-962 (1988)).

*SDS-PAGE and Western Blot* - For western blot experiments cells were cultured 24 hours with or without growth factor(s). Syndecan ectodomain containing material released from the cell surface by trypsin treatment was fractionated on SDS-PAGE gradient (2-15%) gel (O'Farrel, *J. Biol. Chem. 250*:4007-4021 (1975)). After electrophoresis, samples were transferred onto Zeta-Probe membrane using electroblotting 2005 Transphor apparatus (LKB). The syndecan antigen on the filter was detected with radioiodinated mAB 281-2 and the filter was washed, as described above for slot blot analysis.

*Northern Blot* - RNA was Isolated from 3T3 and NMuMG cells using 4 M guanidine isothiocyanate and CsCl density centrifugatlon (Chirgwin *et al., Biochemistry 18*:5294-5299 (1979)). RNA samples were size-separated on 1% agarose formaldehyde gel, transferred to GeneScreen Plus™ membrane (New England Nuclear) and hybridized with multi-prime (Amersham) labeled partial cDNA clone for mouse syndecan (PM-4) (Saunders *et al., J. Cell Biol. 108*:1547-1556 (1989)). After hybridization the membrane was washed in 2 x SSC and 1.0% SDS at 65°C (high stringency conditions). For rehybridization with glyceraldehyde-3-phosphate-dehydrogenase (GAPDH; Fort *et al., Nucleic Acid Res. 13*:1431-1442 (1985)) the bound PM-4 probe was removed as recommended by the manufacturer of the filter (NEN).

### EXAMPLE III

### Induction of syndecan mRNA expression in the human breast cancer cells (MCF-7) growth-inhibited with toremifene.

The steroid-responsive human breast cancer cell line MCF-7 was used to study the expression of human syndecan under different growth conditions regulated by estrogen and antiestrogen. For the experiment cells were plated at a density of 1.2 x 10⁶ cells/100 mm ⌀ plastic culture dishes and grown as monolayer cultures in 10 ml per dish of phenol red-free DMEM medium with 5% dextran/charcoal stripped fetal calf serum (DS-FCS), 2 mM L-glutamine and 3 µg/ml insulin. For hormone-treatment 1 nM 17β-*O*-estradiol (E₂), alone or with 1-6.25 µM toremifene, dissolved 70% in ethanol, were added to the culture medium on the day following plating. The cells were cultured for 6 days, and the media were changed every second day. For RNA extraction the cells were washed *in situ* with PBS and scraped from the plates in 4 M guanidine isothiocyanate.

### EXAMPLE IV

### Treatment of Steroid-Responsive Tumors in Patients.

Patients diagnosed as having a steroid-responsive tumor selected from a breast tumor, an endometrium tumor, a prostate gland tumor or a mesenchymal tissue tumor are administered a composition that contains efficacious amounts of the anti-steroid agent toremifene or tamoxifen, or efficacious amounts of the growth factor bFGF, TGF-β or bFGF together with TGF-β, in amounts ranging from 20-600 mg per day, depending upon the extent of the tumor, the patient's age, the patient's sex, and other treatments such as are taken into consideration in designing such chemotherapeutic protocols. The syndecan-inducing agent is administered for a period of time sufficient to induce syndecan in the tumor cells, such that the tumor cells now take upon a more differentiated phenotype and such that the growth of the tumor is arrested or significantly slowed by the treatment.

### EXAMPLE V

### Stimulation of Hair Growth in Epidermal Skin Cells.

Patients diagnosed as being in need of increased hair growth in the scalp region are administered a composition that contains efficacious amounts of the anti-steroid agent toremifene or tamoxifen, or efficacious amounts of the growth factor bFGF, TGF-β or bFGF together with TGF-β, in amounts ranging from 20-600 mg per day, depending upon the extent of the needed hair growth, the patient's age, the patient's sex, and other treatments such as are taken into consideration in designing such protocols. The syndecan-inducing agent is administered for a period of time sufficient to induce syndecan in the epidermal cells, such that hair growth is significantly increased by the treatment.

### EXAMPLE VI

### Dermination of Mouse Syndecan Promoter and Enhancer Activities

The mouse syndecan gene has been cloned and characterized up to -10 kbs upstream from the transcription start site. To determine the specific activities of different proximal promoter regions (up to -2 kbs from the start site) and enhancer regions (from -2 to -10 kbs) we have made plasmid constructs where these regions were cloned into pCAT basic or pCAT promoter vectors, containing the CAT reporter gene. The reporter CAT gene produces chloramphenicol acetyltransferase enzyme, which transfers the n-butyryl moiety of n-butyryl CoA to chloramphenicol. The n-butyryl chloramphenicol can be separated from native chloramphenicol by xylene.

For the further characterization of the proximal syndecan promoter a series of retriction enzyme treatments was made on the upstream region (Hind III, Hind II, Bgl II, Stu I, Dra I, Cla I, BamHI and Pst I -- Xho I) and the obtained fragments were cloned into the pCAT basic vector. For enhancer areas, three Xba I fragments were cloned into a pCAT promoter vector, where the SV 40 promoter was displaced by the Bgl II - Xho I fragment from the syndecan promoter.

The plasmid constructs were transiently transfected into eukaryotic cells by calcium phosphate precipitation simultaneously with a β-Galactosidase expressing vector to determine the transfection efficiency. After a four hour incubation cells were treated with 15% glycerol and grown for approximately 48 h in cell culture medium. Cells were then scraped from dishes in TEN-buffer and the cytoplasmic extract was obtained by repeated freezing and thawing. β-Galactosidase acitivity was obtained in hte cytoplasmic extreact by adding 0.1 M sodium phosphate, 45 mM mercaptoethanol and 0.2 mg ONPG. This was incubated from 2 hours to overnight and the colour reaction was measured spectrofotometrically at 420 nm.

The CAT activity was determined by adding 0.25 M Tris buffer, 25 ng n-butyryl CoA and 0.0626 µCi of ¹⁴C-chloramphenicol to the cytoplasmic extract. These were incubated overnight, backextracted wiht xylene and radioactivity was measured by scintillation counting. The CAT activity was corrected for transfection efficiency by β-galactosidase activity.

The cells used for proximal promoter constructs were 3T3 NIH, S115 (either hormone-treated of not) and nMuMG cells. For enhancer constructs we used 3T3 NIH cells grown in 2% CMS medium and to test the effect of growth factors in 2% CMS medium with 10 ng/ml FGF-2 and 2ng/ml TGFβ-1.

All references cited herein are fully incorporated herein by reference. Having now fully described the invention, it will be understood by those with skill in the art that the scope may be performed within a wide and equivalent range of conditions, parameters and the like, without affecting the spirit or scope of the invention or any embodiment thereof.

## Claims

1. A nucleic acid molecule comprising the enhancer element of the syndecan gene as represented by SEQ ID NO: 3.

2. The nucleic acid molecule of claim 1 having the sequence of nucleotides of SEQ ID NO: 3.

3. A host cell transfected with a vector comprising the nucleic acid molecule according to claim 1 or 2.

4. A composition containing the nucleic acid molecule according to claims 1 or 2.

5. Use of the nucleic acid molecule according to claims 1 or 2 for the preparation of a pharmaceutical composition for decreasing the growth of a malignant tumor in a patient.

## Patentansprüche

1. Nucleinsäuremolekül, umfassend das Enhancer-Element des Syndecan-Gens wie in Seq. ID Nr. 3 dargestellt.

2. Nucleinsäuremolekül nach Anspruch 1 mit der Nucleotidsequenz von Seq. ID Nr. 3.

3. Wirtszelle, die mit einem Vektor transfiziert ist, der das Nucleinsäuremolekül nach Anspruch 1 oder 2 umfaßt.

4. Zusammensetzung, die das Nucleinsäuremolekül nach Anspruch 1 oder 2 enthält.

5. Verwendung des Nucleinsäuremoleküls nach Anspruch 1 oder 2 für die Herstellung eines Arzneimittels zur Verminderung des Wachstums eines malignen Tumors in einem Patienten.

## Revendications

1. Molécule d'acide nucléique comprenant l'élément amplificateur du gène du syndécane tel que représenté par SEQ ID NO: 3.

2. Molécule d'acide nucléique selon la revendication 1 ayant la séquence de nucléotides de SEQ ID NO: 3.

3. Cellule hôte transfectée par un vecteur comprenant la molécule d'acide nucléique selon la revendication 1 ou 2.

4. Composition contenant la molécule d'acide nucléique selon la revendication 1 ou 2.

5. Utilisation de la molécule d'acide nucléique selon la revendication 1 ou 2 pour la préparation d'une composition pharmaceutique destinée à diminuer la croissance d'une tumeur maligne chez un patient.
